# EUROPEAN PATENT APPLICATION

(11) **EP 4 335 925 A2**
(43) Date of publication of application: **13.03.2024**
(21) Application number: 23211261.5
(22) Date of filing: 09.04.2020
(51) Int. Cl.: C12N 15/86

(54) **LONG-LASTING ANALGESIA VIA TARGETED IN VIVO EPIGENETIC REPRESSION**

(30) Priority: 09.04.2019 US 201962831706 P; 23.07.2019 US 201962877810 P
(62) Divisional of application: 20787604.6
(71) Applicant: The Regents of the University of California, Oakland, CA 94607-5200 (US)
(72) Inventor: MALI, Prashant, La Jolla, CA 92093 (US); MORENO, Ana, San Diego, CA 92103 (US)
(74) Representative: Gill Jennings & Every LLP

(57) **Abstract**

The disclosure provides epigenetic based approaches and methods using genome editing constructs comprising a zinc finger fused with a repressor domain and/or a dCas9 fused with a repressor domain to treat and manage pain in subjects in need of treatment thereof.

## Description

### CROSS REFERENCE TO RELATED APPLICATIONS

This application claims priority under 35 U.S.C. §119 from Provisional Application Serial No. 62/831,706, filed April 9, 2019 and from Provisional Application Serial No. 62/877,810, filed July 23, 2019, the disclosures of which are incorporated herein by reference in their entireties for all purposes.

### STATEMENT REGARDING FEDERALLY SPONSORED RESEARCH

This invention was made with government support under CA222826, GM123313, and HG009285 awarded by the National Institutes of Health. The government has certain rights in the invention.

### TECHNICAL FIELD

The disclosure provides epigenetic based approaches and methods using genome editing constructs comprising a zinc finger fused with a repressor domain and/or a dCas9 fused with a repressor domain to treat and manage pain in subjects in need of treatment thereof.

### INCORPORATION BY REFERENCE OF SEQUENCE LISTING

Accompanying this filing is a Sequence Listing entitled "Sequence-Listing_ST25.txt", created on April 9, 2020 and having 121,918 bytes of data, machine formatted on IBM-PC, MS-Windows operating system. The sequence listing is incorporated herein by reference in its entirety for all purposes.

### BACKGROUND

Chronic pain affects between 19% to 50% of the world population, with more than 100 million people affected in the U.S. alone. Moreover, the number of people reporting chronic pain is expected to increase by 2035 due to the aging global population and prevalence of chronic diseases. While chronic pain is more prevalent than cancer, diabetes and cardiovascular disease combined, drug development has not undergone the remarkable progress seen in these other therapeutic areas. Furthermore, current standard of care for chronic pain often relies on opioids, which can have adverse side effects and significant addiction risk. Despite decades of research, the goal of achieving broadly effective, long-lasting, non-addictive therapeutics for chronic pain has remained elusive.

### SUMMARY

The disclosure provides recombinant gene repressor complex comprising a nuclease inactivated Cas9 (dCas9) protein fused to a transcription repressor and associated with at least one guide RNA (gRNA), wherein the gRNA specifically hybridizes to a target nucleic acid sequence encoding a gene product selected from the group consisting of TRPV1/2/3/4, P2XR3, TRPM8, TRPA1, P23X2, P2RY, BDKRB1/2, Hlr3A, ACCNs, TRPV4, TRPC/P, ACCN1/2, SCN1/3/8A/9A, SCN10A, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited. In one embodiment, the target nucleic acid sequence is located on chromosome 2 at position 2q24.3. In another or further embodiment, the gRNA comprises a sequence encoded by the sequence set forth in any one of 11-107. In another or further embodiment the gRNA specifically hybridizes to a nucleic acid sequence encoding a SCN9A product (Nav1.7). In another or further embodiment, the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB). In another embodiment, the transcriptional repressor domain is a KRAB domain.

The disclosure also provides a polynucleotide encoding one or more components of the recombinant gene repressor complex described above and herein. In one embodiment, the polynucleotide is codon optimized for expression in a human cell.

The disclosure also provides a vector or vector system comprising a polynucleotide of the disclosure encoding one or more components to generate a recombinant gene repressor complex of the disclosure. In one embodiment, the polynucleotide is operably linked to a promoter. In another embodiment, the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/calmodulin-dependent protein kinase II promoter, a tubulin alpha I promoter, a neuron-specific enolase promoter and a glial fibrillary acidic protein (GFAP) promoter. In yet another embodiment, the vector comprises a polIII promoter upstream of the at least one guide RNA coding sequence. In a further embodiment, the polIII promoter is selected from a U6 and H1 promoter. In another embodiment, the vector further comprises a regulatory control sequence. In a further embodiment, the regulatory control sequence is a woodchuck hepatitis virus posttranscriptional regulatory element (WPRE). In another embodiment, the vector is a recombinant adeno-associated virus vector (rAAV vector). In a further embodiment, the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV). In still another or further embodiment, the polynucleotide includes one or more inverted repeats (ITRs). In still another or further embodiment, the polynucleotide includes a poly A sequence. In another embodiment, the vector and polynucleotide are engineered to be expressed in a cell. In yet another embodiment, the vector is a lentiviral vector, a gammaretroviral vector, or a herpes simplex viral vector. In still another embodiment, the vector comprises a split dCas9 vector system. In another embodiment, the vector comprises a nucleic acid encoding a dCas9 having a sequence as set forth in SEQ ID NO:2 or a sequence that is at least 90% identical thereto and can complex with gRNA and lacks nuclease activity. In still another embodiment, the vector comprises a nucleic acid encoding a KRAB sequence of SEQ ID NO:7 or a sequence that is at least 90% identical thereto and can repress transcription. In yet another embodiment, the split vector system comprises a vector sequence selected from SEQ ID NO: 3, 4 and 10 or sequences that are at least 90% to 99% identical thereto.

The disclosure provides a zinc-finger repressor construct comprising an engineered zinc finger DNA-binding domain coupled to a transcription repressor, wherein the zinc finger DNA-binding domain comprises one to six zinc-finger sequences and wherein the zinc finger sequences bind to a target nucleic acid sequence in a gene encoding a gene product selected from the group consisting of TRPV1/2/3/4, P2XR3, TRPM8, TRPA1, P23X2, P2RY, BDKRB1/2, Hlr3A, ACCNs, TRPV4, TRPC/P, ACCN1/2, SCN1/3/8A/9A, SCN10A, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited. In one embodiment, the target nucleic acid sequence is a sequence set forth in Table 2. In still another embodiment, the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).

The disclosure also provides a polynucleotide encoding the zinc-finger repressor construct described above and herein. In one embodiment, the polynucleotide is codon optimized for expression in a human cell.

The disclosure also provides a vector containing the polynucleotide encoding a zinc finger repressor construct of the disclosure. In one embodiment, the polynucleotide is operably linked to a promoter. In a further embodiment, the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/calmodulin-dependent protein kinase II promoter, a tubulin alpha I promoter, a neuron-specific enolase promoter and a glial fibrillary acidic protein (GFAP) promoter. In another embodiment, the vector further comprise a regulatory control sequence. In a further embodiment, the regulatory control sequence is a woodchuck hepatitis virus posttranscriptional regulatory element (WPRE). In another embodiment, the vector is a recombinant adeno-associated virus vector (rAAV vector). In a further embodiment, the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV). In another embodiment, the Vector or polynucleotide includes one or more inverted repeats (ITRs). In another embodiment, the vector or polynucleotide includes a poly A sequence. In yet another embodiment, the nucleic acid is engineered to express the one or more components in a cell. In another embodiment, the vector is a lentiviral vector, a gammaretroviral vector, or a herpes simplex viral vector. In another embodiment, the vector comprises a nucleic acid encoding a KRAB sequence of SEQ ID NO:7 or a sequence that is at least 90% to 99% identical thereto.

The disclosure also provides an epigenetic-based method to treat or manage chronic pain in a subject comprising administering an effective amount of a complex or a construct as described herein and above.

The disclosure also provides an epigenetic-based method to treat or manage pain in a subject in need thereof, comprising administering an effective amount of a zinc finger-repressor construct and/or a dCas9-repressor domain complex to the subject, wherein dCas9 is catalytically inactivated Cas9 that does not cleave DNA but maintains its ability to bind to the genome via a guide-RNA (gRNA). In one embodiment, the pain is selected from neuropathic pain, nociceptive pain, allodynia, inflammatory pain, inflammatory hyperalgesia, neuropathies, neuralgia, diabetic neuropathy, human immunodeficiency virus-related neuropathy, nerve injury, rheumatoid arthritic pain, osteoarthritic pain, burns, back pain, eye pain, visceral pain, cancer pain, bone cancer pain, migraine pain, pain from carpal tunnel syndrome, fibromyalgia pain, neuritis pain, sciatica pain, pelvic hypersensitivity pain, pelvic pain, post herpetic neuralgia pain, post-operative pain, post-stroke pain, and menstrual pain. In another embodiment, the pain is associated with a disease or disorder selected from the group consisting of neuropathic peripheral neuropathy, diabetic neuropathy, post herpetic neuralgia, trigeminal neuralgia, back injury, cancer neuropathy, HIV neuropathy, limb loss, carpal tunnel syndrome, stroke, alcoholism, hypothyroidism, uremia, multiple sclerosis, spinal cord injury, Parkinson's disease, and epilepsy. In another embodiment, the method is used to treat a subject with chronic pain. In still another embodiment, the zinc finger-repressor construct comprises a repressor domain selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB). In a further embodiment, the repressor domain comprises KRAB. In another embodiment, the zing finger-repressor construct binds to a target of Table 2. In another embodiment, the dCas9-repressor domain complex comprises a repressor domain selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB). In a further embodiment, the repressor domain comprises KRAB. In another embodiment, the dCas9-repressor domain construct comprises a guide RNA spacer sequence having a sequence selected from SEQ ID NOs:11-106 and 107. In still another embodiment, the zinc finger-repressor construct and/or the dCas9-repressor domain construct provides for non-permanent gene repression of a voltage gated sodium channel. In a further embodiment, the voltage gated sodium channel is selected from NaV1.7, NaV1.8, and NaV1.9. In still a further embodiment, the voltage gated sodium channel is NaV1.7. In another embodiment, the zinc finger-repressor construct and/or the dCas9-repressor domain construct is packaged and delivered by a recombinant virus. In a further embodiment, the recombinant virus is an adenovirus, gammaretrovirus, adeno-associated virus (AAV), herpes simplex virus (HSV) or lentivirus. In a further embodiment, the recombinant virus is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV). In another embodiment, the zinc finger-repressor construct and/or the dCas9-repressor domain construct is administered intravenous, intraperitoneal, intrathecal, intraganglionic, intraneural, intracranial or intramuscular.

### DESCRIPTION OF DRAWINGS

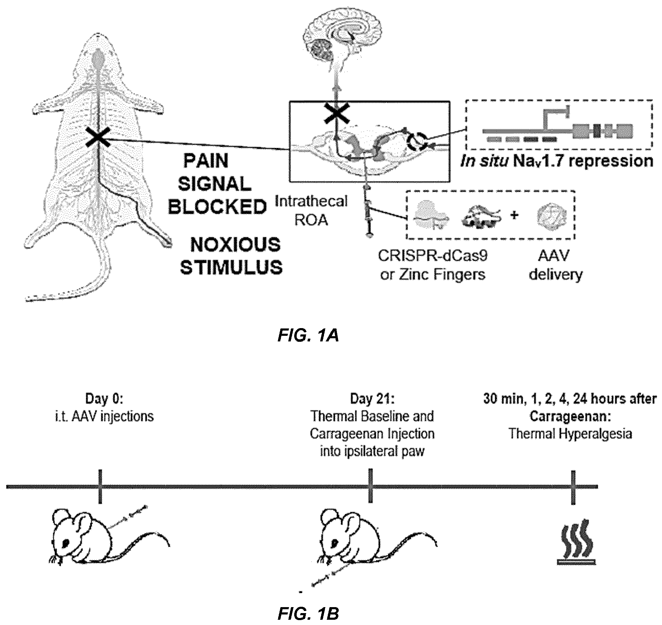

Figure 1A-D shows *in situ* repression of NaV1.7 leads to pain amelioration in a carrageenan model of inflammatory pain. (**A**) Schematic of the overall strategy used for *in situ* NaV1.7 repression using ZFP-KRAB and KRAB-dCas9. NaV1.7 is a DRG channel involved in the transduction of noxious stimuli into electric impulses at the peripheral terminals of DRG neurons. *In situ* repression of NaV1.7 via AAV-ZFP-KRAB and AAV-KRAB-dCas9 is achieved through intrathecal injection leading to disruption of the pain signal before reaching the brain. (**B**) Schematic of the carrageenan-induced inflammatory pain model. At day 0, mice were intrathecally injected with either AAV9-Zinc-Finger-4-KRAB, AAV9-mCherry, AAV9-KRAB-dCas9-dual-gRNA or AAV9-KRAB-dCas9-no-gRNA. 21 days later, thermal pain sensitivity was measured in all mice with the Hargreaves assay. In order to establish a baseline level of sensitivity, mice were tested for tactile threshold using von Frey filaments before carrageenan injection. Mice were then injected with carrageenan in their left paw (ipsilateral) while the right paw (contralateral) was injected with saline as an in-mouse control. They were then tested for thermal paw-withdrawal latency at 30 min, 1, 2, 4, and 24 hours after carrageenan administration. (**C**) *In vivo* NaV1.7 repression efficiencies: Twenty-four hours after carrageenan administration, mice DRG (L4-L6) were harvested and NaV1.7 repression efficacy was determined by qPCR. (n=5; error bars are SEM; Student's t-test; ***p = 0.0008, **p=0.0033). (**D**) The aggregate paw withdrawal latency was calculated as area under the curve (AUC) for both carrageenan and saline injected paws. Mice treated with Zinc-Finger-4-KRAB and KRAB-dCas9-dual-gRNA had significant increased paw-withdrawal latencies in carrageenan-injected paws (n=10; error bars are SEM; Student's t-test, ****p < 0.0001).

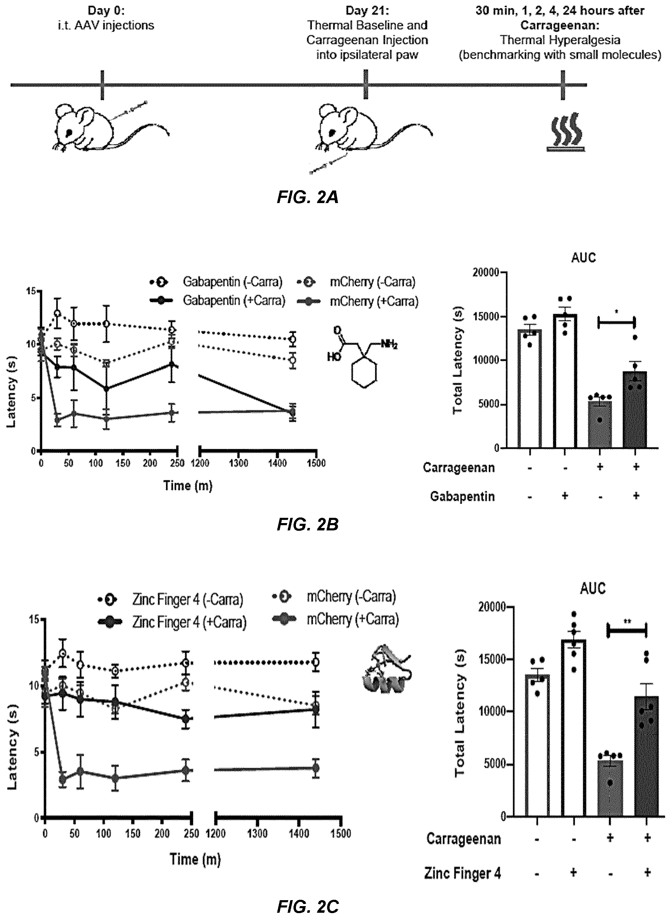

Figure 2A-C shows benchmarking of *in situ* repression of NaV1.7 using Zinc-Finger-KRAB with established small molecule drug gabapentin. (**A**) Schematic of the experimental approach. (**B-C**) Time course of thermal hyperalgesia after the injection of carrageenan (solid lines) or saline (dotted lines) into the hind paw of mice injected with gabapentin (100mg/kg) and mCherry and Zinc-Finger-4-KRAB are plotted. Mean paw withdrawal latencies (PWL) are shown. The AUC of the thermal-hyperalgesia time-course are plotted on the right panels. A significant increase in PWL is seen in the carrageenan-injected paws of mice injected with gabapentin and Zinc-Finger-4-KRAB (n=5 for mCherry and gabapentin and n=6 for Zinc-Finger-4-KRAB; error bars are SEM; Student's t-test, *p = 0.0208, **p=0.0021).

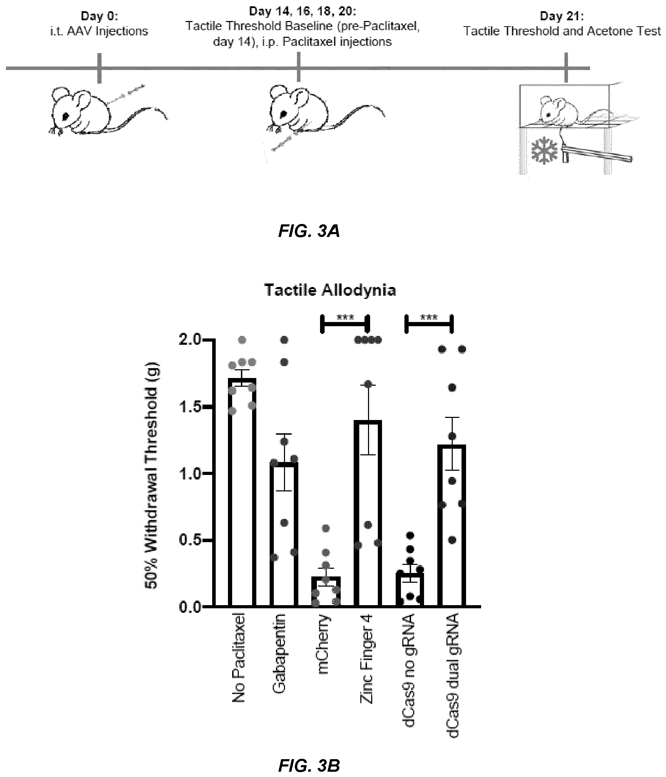

Figure 3A-E shows *in vivo* efficacy of Zinc-Finger-KRAB and KRAB-dCas9 in two neuropathic pain models (**A**) Schematic of the paclitaxel-induced neuropathic pain model. Mice were i.t. injected with AAV9-mCherry, AAV9-Zinc-Finger-4-KRAB, AAV9-KRAB-dCas9-no-gRNA, AAV9-KRAB-dCas9-dual-gRNA or saline. Following baseline von Frey threshold testing at day 14, mice were then injected i.p. with 8mg/kg of paclitaxel at 14, 16, 18, 20 days after i.t. injection. 21 days after i.t. injection, mice were tested for tactile allodynia via von Frey filaments and for cold allodynia via the application of acetone. (**B**) *In situ* repression of NaV1.7 via Zinc-Finger-4-KRAB and KRAB-dCas9-dual-gRNA reduces paclitaxel-induced tactile allodynia. (n=8; error bars are SEM; Student's t-test; ***p = 0.0007, ***p = 0.0004) (**C**) *In situ* repression of NaV1.7 via Zinc-Finger-4-KRAB and KRAB-dCas9-dual-gRNA reduces paclitaxel-induced cold allodynia. (n=8; error bars are SEM; Student's t-test; ****p < 0.0001, **p = 0.008). (D) Schematic of the BzATP pain model. Mice were injected at day 0 with AAV9-mCherry, AAV9-KRAB-dCas9-no-gRNA or AAV9-KRAB-dCas9-dual-gRNA. 21 days later, mice were baselined for von Frey tactile threshold and were then i.t. injected with 30 nmol BzATP. 30 minutes, 1, 2, 3, 6, and 24 hours after BzATP administration, mice were tested for tactile allodynia. (E) *In situ* repression of Nav1.7 via KRAB-dCas9-dual-gRNA reduces tactile allodynia in a BzATP model of neuropathic pain (n=5 for KRAB-dCas9-no-gRNA and n=6 for the other groups, two-way ANOVA with Bonferonni post-hoc test; ****p < 0.0001, *p = 0.0469).

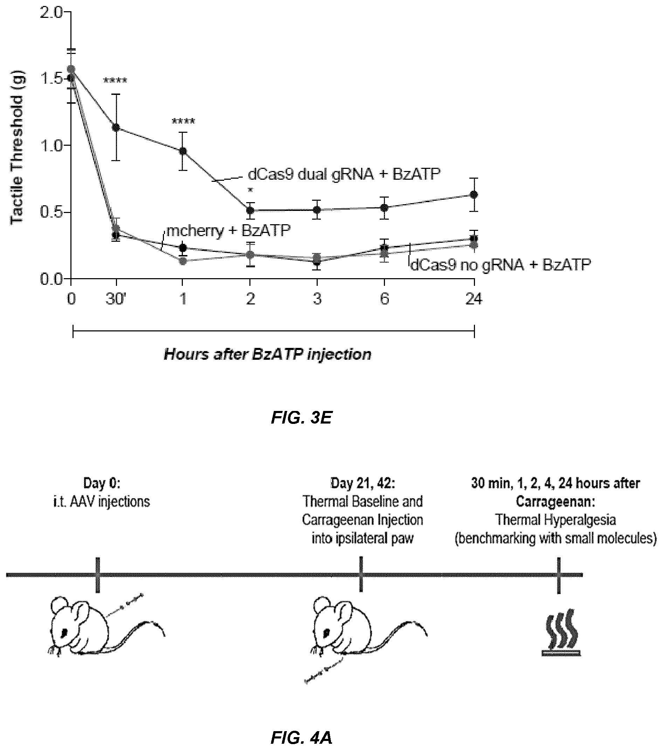

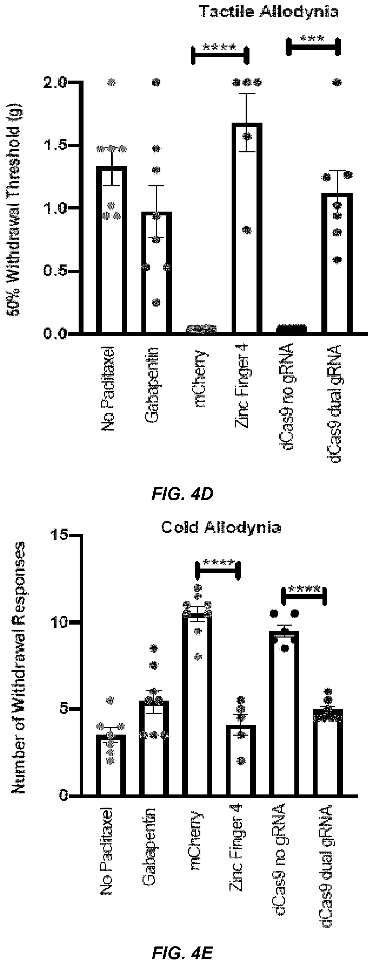

Figure 4A-E shows long-term efficacy of Zinc-Finger-KRAB and KRAB-dCas9 in two independent pain models. (**A**) Timeline of the carrageenan-induced inflammatory pain model. (**B**) Time course of thermal hyperalgesia after the injection of carrageenan (solid lines) or saline (dotted lines) into the hind paw of mice 42 days after i.t. injection with AAV9-mCherry and AAV9-Zinc-Finger-4-KRAB are plotted. Mean paw withdrawal latencies are shown. The AUC of the thermal-hyperalgesia time-course are plotted on the right panel. A significant increase in PWL is seen in the carrageenan-injected paws of mice injected with AAV9-Zinc-Finger-4-KRAB (n=8; error bars are SEM; Student's t-test; ****p < 0.0001). (**C**) Schematic of the paclitaxel-induced neuropathic pain model. (**D**) *In situ* repression of NaV1.7 via Zinc-Finger-4-KRAB and KRAB-dCas9-dual-gRNA reduces paclitaxel-induced tactile allodynia 49 days after last paclitaxel injection (n=7 for Zinc-Finger-4-KRAB and n=8 for other groups; error bars are SEM; Student's t-test; ****p < 0.0001). (**E**) *In situ* repression of NaV1.7 via Zinc-Finger-4-KRAB and KRAB-dCas9-dual-gRNA reduces paclitaxel-induced cold allodynia. (n=7 for Zinc-Finger-4-KRAB and n=8 for other groups; error bars are SEM; Student's t-test; ****p < 0.0001, ***p = 0.0001).

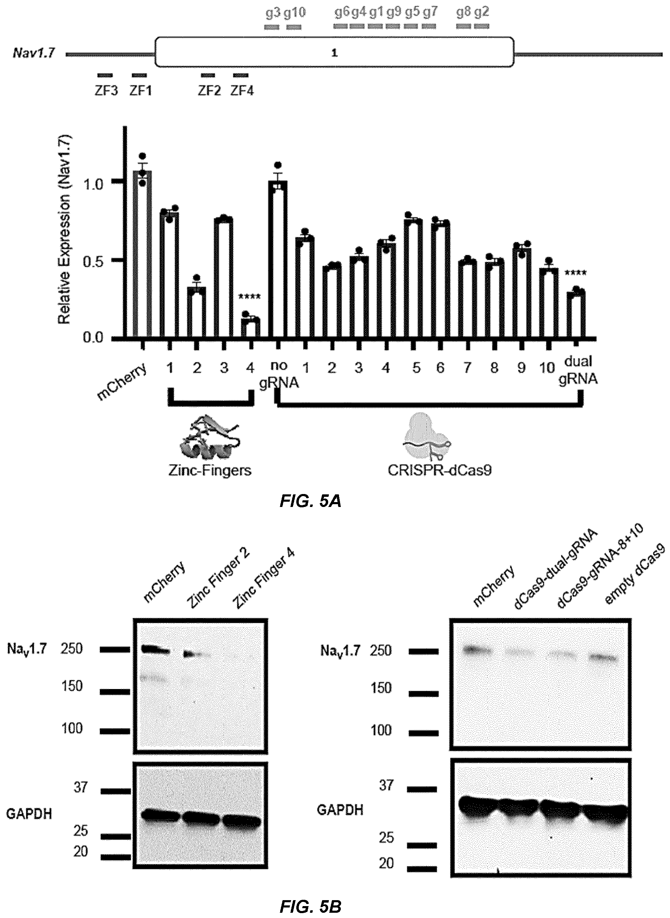

Figure 5A-B shows *in vitro* optimization of epigenetic genome engineering tools to enable NaV1.7 repression. (**A**) A panel of four zinc finger proteins and ten gRNAs were designed to target NaV1.7 in a mouse neuroblastoma cell line (Neuro2a) and were screened for repression efficacy by qPCR. A non-targeting gRNA (no gRNA) was used as a control for KRAB-dCas9 constructs targeting NaV1.7, while mCherry was used as a control for ZFP-KRAB constructs targeting NaV1.7 (n=3; error bars are SEM; one-way ANOVA; ****p < 0.0001). (**B**) *In vitro* western blotting of NaV1.7 in Neuro2a cells transfected with mCherry, Zinc-Finger-2-KRAB, Zinc-Finger-4-KRAB, KRAB-dCas9-no-gRNA, KRAB-dCas9-dual-gRNA (1+2), and KRAB-dCas9-gRNA-8+10.

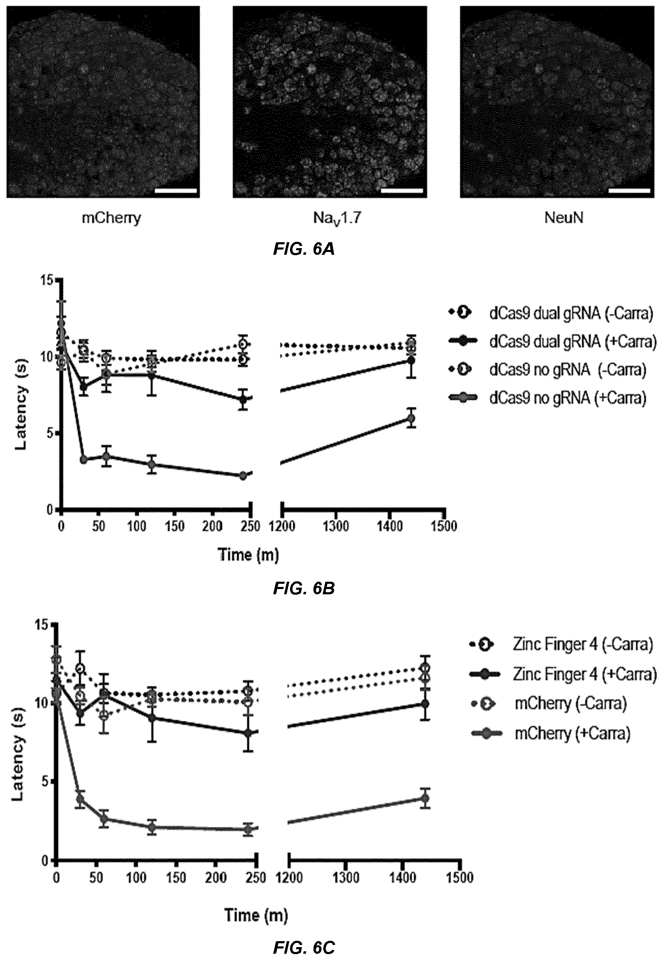

Figure 6A-D shows *in situ* repression of NaV1.7 leads to pain amelioration in a carrageenan model of inflammatory pain. (**A**) Confirming AAV9-mCherry transduction in mice DRG via RNA FISH (red= mCherry, pink= NaV1.7, green= NeuN; scale bar=50µm). (**B**) Time course of thermal hyperalgesia after the injection of carrageenan (solid lines) or saline (dotted lines) into the hind paw of mice 21 days after i.t. injection with AAV9-KRAB-dCas9-no-gRNA and AAV9-KRAB-dCas9-dual-gRNA are plotted. Mean paw withdrawal latencies are shown. (n=10; error bars are SEM). (**C**) Time course of thermal hyperalgesia after the injection of carrageenan (solid lines) or saline (dotted lines) into the hind paw of mice 21 days after i.t. injection with AAV9-mCherry and AAV9-Zinc-Finger-4-KRAB are plotted. Mean paw withdrawal latencies are shown. (n=10; error bars are SEM). (**D**) Paw thickness of ipsilateral paws at baseline and four hours after carrageenan injection are plotted (n=10).

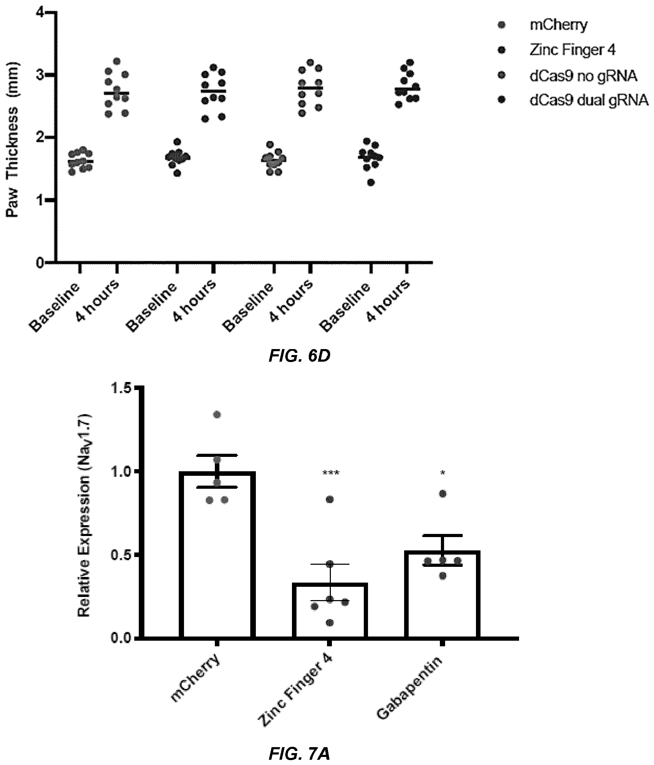

Figure 7A-D shows evaluation of Zinc-Finger-KRAB in an inflammatory model of pain. (**A**) *In vivo* NaV1.7 repression efficiencies from treated mice DRG. Twenty-four hours after carrageenan administration, mice DRG (L4-L6) were harvested and NaV1.7 repression efficacy was determined by qPCR. (n=5 for mCherry and Gabapentin groups and n=6 for Zinc-Finger-4-KRAB group; error bars are SEM; one way ANOVA with Dunnet's post hoc test; ***p = 0.0007, *p=0.0121). (**B**) Paw thickness of ipsilateral paws at baseline and four hours after carrageenan injection are plotted. (**C**) Significance of paw withdrawal latencies in mice receiving AAV9-Zinc-Finger-4-KRAB and gabapentin (100 mg/kg) as compared to AAV9-mCherry carrageenan-injected paw (negative control). Two-way ANOVA with Bonferroni post hoc test. (**D**) Independent repeat of experiment in (a): time course of thermal hyperalgesia after the injection of carrageenan (solid lines) or saline (dotted lines) into the hind paw of mice 21 days after i.t. injection with AAV9-mCherry and AAV9-Zinc-Finger-4-KRAB are plotted. Mean paw withdrawal latencies are shown. The AUC of the thermal-hyperalgesia time-course are plotted on the right panel. A significant increase in PWL is seen in the carrageenan-injected paws of mice injected with AAV9-Zinc-Finger-4-KRAB (n=8; error bars are SEM; Student's t-test; ****p < 0.0001).

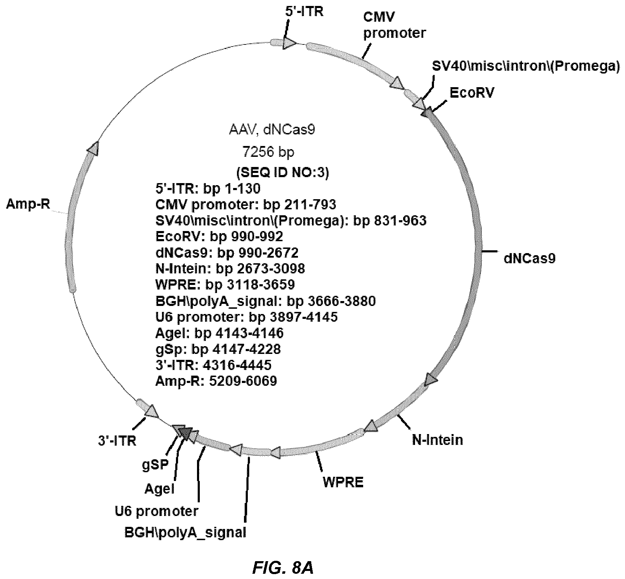

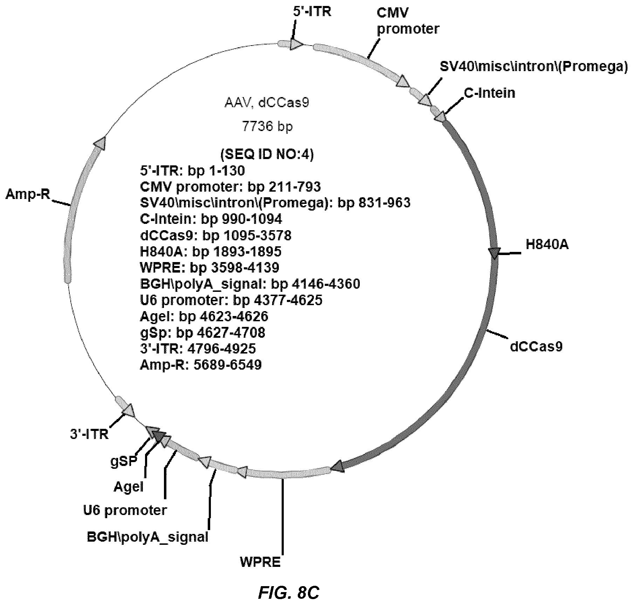

Figure 8A-C shows plasmid constructs used in the methods and compositions of the disclosure. (**A**) provides a plasmid construct for AAV,dNCas9 (SEQ ID NO:3); (**B**) provides a plasmid construct for AAV,dNCas9-KRAB (SEQ ID NO:10); and (**C**) provides a plasmid construct for AAV,dCCas9 (SEQ ID NO:4).

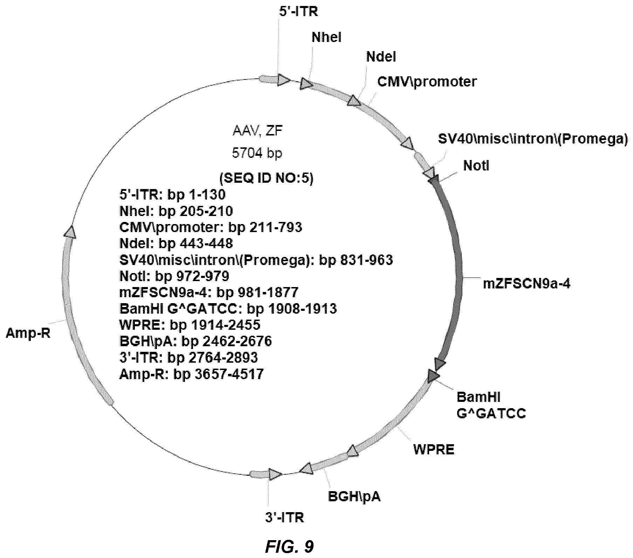

**Figure** 9 shows the zinc-finger plasmid construct used in the methods and compositions of the disclosure (SEQ ID NO:5).

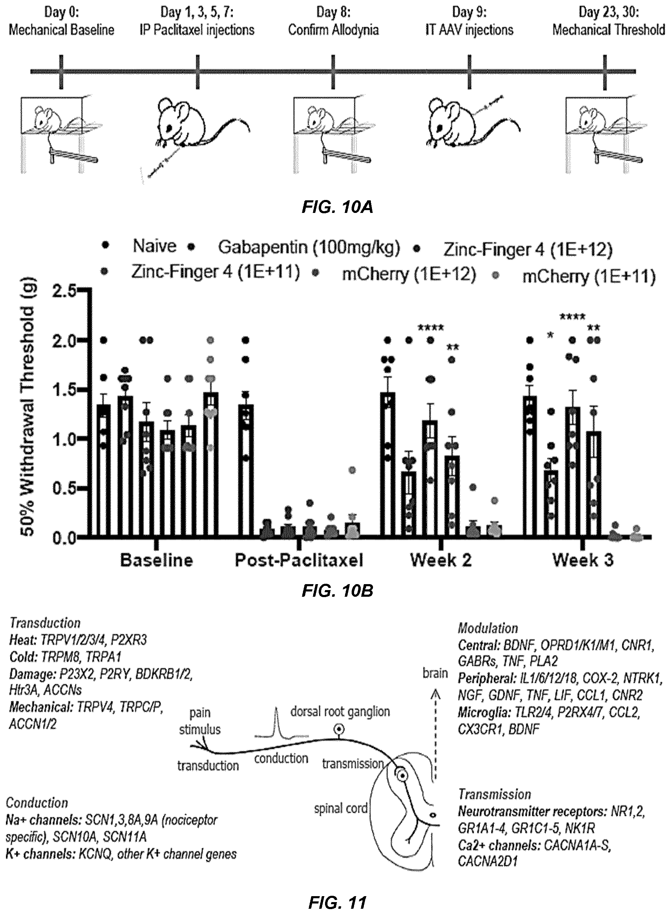

Figure 10A-B show *in situ* repression of NaV1.7 can reverse chemotherapy-induced neuropathic pain. (**A**) Schematic of the treatment for paclitaxel-induced chronic neuropathic pain model. In order to establish a baseline level of sensitivity, mice were tested for tactile threshold using von Frey filaments. Mice were then injected i.p. with 8mg/kg of paclitaxel at days 1, 3, 5, and 7. After confirming tactile allodynia via von Frey filaments, mice were intrathecally injected at day 9 with either (1E+11 or 1E+12 vg/mouse) AAV9-Zinc-Finger-4-KRAB or AAV9-mCherry (1E+11 or 1E+12 vg/mouse). 14 and 21 days later, mice were tested for tactile allodynia via von Frey filaments. (**B**) *In situ* repression of NaV1.7 via Zinc-Finger-4-KRAB reduces paclitaxel-induced tactile allodynia. (n=8; error bars are SEM; Student's t-test; week 2 ****p < 0.0001, *p = 0.0029; week 3 ****p < 0.0001, ***p = 0.0012.
**Figure 11** provides a curated list of genes involved in sensing of pain, highlighting also the multiple modes of potential therapeutic intervention.

### DETAILED DESCRIPTION

As used herein and in the appended claims, the singular forms "a," "an," and "the" include plural referents unless the context clearly dictates otherwise. Thus, for example, reference to "a zinc finger" includes a plurality of such zinc fingers and reference to "the Adeno-associated virus" includes reference to one or more Adeno-associated viruses and equivalents thereof known to those skilled in the art, and so forth.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

Unless defined otherwise, all technical and scientific terms used herein have the same meaning as commonly understood to one of ordinary skill in the art to which this disclosure belongs. Although many methods and reagents are similar or equivalent to those described herein, the exemplary methods and materials are disclosed herein.

All publications mentioned herein are incorporated herein by reference in full for the purpose of describing and disclosing the methodologies, which might be used in connection with the description herein. Moreover, with respect to any term that is presented in one or more publications that is similar to, or identical with, a term that has been expressly defined in this disclosure, the definition of the term as expressly provided in this disclosure will control in all respects.

"Chronic pain," as used herein, means pain that is marked by a duration and/or frequency of recurrence that excludes acute pain of only limited duration and without recurrence. In some cases, "chronic pain" persists for a duration of six months or more, or longer than the temporal course of natural healing processes that may otherwise be associated with a particular injury, condition or disease. "Chronic pain" includes, without limitation, neuropathic pain, inflammatory pain, cancer pain, thermal pain and mechanical pain, or a combination of two or more of the foregoing.

The term "encode" as it is applied to nucleic acid sequences refers to a polynucleotide which is said to "encode" a polypeptide if, in its native state or when manipulated by methods well known to those skilled in the art, can be transcribed and/or translated to produce an mRNA for a polypeptide and/or a fragment thereof. The antisense strand is the complement of such a nucleic acid, and the encoding sequence can be deduced therefrom.

The terms "equivalent" or "biological equivalent" are used interchangeably when referring to a particular molecule, biological, or cellular material and intend those having minimal homology while still maintaining desired structure or functionality.

As used herein, the term "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in a eukaryotic cell. The expression level of a gene may be determined by measuring the amount of mRNA or protein in a cell or tissue sample; further, the expression level of multiple genes can be determined to establish an expression profile for a particular sample.

As used herein, the term "functional" may be used to modify any molecule, biological, or cellular material to intend that it accomplishes a particular, specified effect.

A "fusion molecule" is a molecule in which two or more subunit molecules are linked, typically covalently. The subunit molecules can be the same chemical type of molecule, or can be different chemical types of molecules. Examples of the first type of fusion molecule include, but are not limited to, fusion polypeptides (for example, a fusion between a ZFP DNA-binding domain and a transcriptional repression domain; or a Cas9 or dCas9 and a transcription repression domain) and fusion nucleic acids (for example, a nucleic acid encoding the fusion polypeptide described supra).

"Gene repression" and "inhibition of gene expression" refer to any process that results in a decrease in production of a gene product. A gene product can be either RNA (including, but not limited to, mRNA, rRNA, tRNA, and structural RNA) or protein. Accordingly, gene repression includes those processes that decrease transcription of a gene and/or translation of an mRNA. Examples of gene repression processes which decrease transcription include, but are not limited to, those which inhibit formation of a transcription initiation complex, those which decrease transcription initiation rate, those which decrease transcription elongation rate, those which decrease processivity of transcription and those which antagonize transcriptional activation (by, for example, blocking the binding of a transcriptional activator). Gene repression can constitute, for example, prevention of activation as well as inhibition of expression below an existing level. Examples of gene repression processes that decrease translation include those that decrease translational initiation, those that decrease translational elongation and those that decrease mRNA stability. Transcriptional repression includes both reversible and irreversible inactivation of gene transcription. In general, gene repression comprises any detectable decrease in the production of a gene product, in some instances a decrease in production of a gene product by about 2-fold, in other instances from about 2- to about 5-fold or any integer there between, in yet other instances between about 5- and about 10-fold or any integer there between, in still other instances between about 10- and about 20-fold or any integer there between, sometimes between about 20- and about 50-fold or any integer there between, in other instances between about 50- and about 100-fold or any integer there between, and in still other instances 100-fold or more. In yet other instances, gene repression results in complete inhibition of gene expression, such that no gene product is detectable.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PC reaction, or the enzymatic cleavage of a polynucleotide. Examples of stringent hybridization conditions include: incubation temperatures of about 25°C to about 37°C; hybridization buffer concentrations of about 6x SSC to about 10x SSC; formamide concentrations of about 0% to about 25%; and wash solutions from about 4x SSC to about 8x SSC. Examples of moderate hybridization conditions include: incubation temperatures of about 40°C to about 50°C; buffer concentrations of about 9x SSC to about 2x SSC; formamide concentrations of about 30% to about 50%; and wash solutions of about 5x SSC to about 2x SSC. Examples of high stringency conditions include: incubation temperatures of about 55°C to about 68°C; buffer concentrations of about lx SSC to about 0.1x SSC; formamide concentrations of about 55% to about 75%; and wash solutions of about lx SSC, 0.1x SSC, or deionized water. In general, hybridization incubation times are from 5 minutes to 24 hours, with 1, 2, or more washing steps, and wash incubation times are about 1, 2, or 15 minutes. SSC is 0.15 M NaCl and 15 mM citrate buffer. It is understood that equivalents of SSC using other buffer systems can be employed.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the disclosure. Methods and algorithms available for determining "identity" or "homology" between two polypeptides or between two nucleic acid molecules are well known in the art and available on-line through the World-Wide-Web.

The term "isolated" as used herein refers to molecules or biologics or cellular materials being substantially free from other materials.

"Nav1.7" (also call SCN9A, ETHA, FEB3B, GEFSP7, HSAN2D, NE-NA, NENA, PN1, SFNP, and sodium voltage-gated channel alpha subunit 9) is a sodium ion channel that in humans is encoded by the SCN9A gene. It is usually expressed at high levels in two types of neurons: the nociceptive (pain) neurons at dorsal root ganglion (DRG) and trigeminal ganglion and sympathetic ganglion neurons, which are part of the autonomic (involuntary) nervous system. The Nav1.7 channel produces a rapidly activating and inactivating current which is sensitive to the level of tetrodotoxin. The sequence and chromosomal location of the SCN9A gene is known.

As used herein, the terms "nucleic acid sequence" and "polynucleotide" are used interchangeably to refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, nonnatural, or derivatized nucleotide bases. In some instances the disclosure provides nucleic acid sequences in the form of a sequence listing. Where the sequence listing provides a DNA sequence, the disclosure further contemplates RNA (*i.e*., wherein 'T" is replaced with 'U' in any of the sequences provided herein.

The term "promoter" as used herein refers to any sequence that regulates the expression of a coding sequence, such as a gene. Promoters may be constitutive, inducible, repressible, or tissuespecific, for example. The term includes mini- and core-promoters of between 50-500 base pairs and includes both polII and polIII promoters. A "promoter" is a control sequence that is a region of a polynucleotide sequence at which initiation and rate of transcription are controlled. It may contain genetic elements at which regulatory proteins and molecules may bind such as RNA polymerase and other transcription factors. Non-limiting exemplary promoters include CMV promoter and U6 promoter. Suitable polIII promoters include, but are not limited to, U6 (mouse and human), H1 (mouse and human). PolIII promoters are suitable for processing small RNA strands (*e.g*. shRNA *etc*.). The disclosure contemplates the use of various promoters (polII) to drive transcription of the CRISPRi, CRISPRi-repressor etc. For example, non-specific promoters can be used such as, but not limited to, CMV, CAG, SV40, RSV, EF1α *etc*. Alternatively cell-specific promoters can be used including, but not limited to, Nav1.7-, Nav1.8-, Nav1.9-specific promoters, TPRV1 promoters etc. In still another embodiment, neuronal-specific promoter can be used including, but not limited to, synapsin I promoters, calcium/calmodulin-dependent protein kinase II promoters, tubulin alpha1 promoters and neuron-specific enolase promoters.

The term "protein", "peptide" and "polypeptide" are used interchangeably and in their broadest sense to refer to a compound of two or more subunits of amino acids, amino acid analogs or peptidomimetics. The subunits may be linked by peptide bonds. In another aspect, the subunit may be linked by other bonds, *e.g.,* ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics.

As used herein, the term "recombinant expression system" refers to a genetic construct for the expression of certain genetic material formed by recombination. Examples of recombinant expression systems include AAV vectors of the disclosure which comprise a number of recombinant domains (see, *e.g.,* FIGs. 8A-C and **9**) for the expression of components of the disclosure.

As used herein, the term "subject" is intended to mean any animal. In some embodiments, the subject may be a mammal; in further embodiments, the subject may be a bovine, equine, feline, murine, porcine, canine, human, or rat.

As used herein, the term "vector" intends a recombinant vector that retains the ability to infect and transduce non-dividing and/or slowly-dividing cells and integrate into the target cell's genome or remain epigenetic. The vector may be derived from or based on a wild-type virus. Aspects of this disclosure relate to an adeno-associated virus vector (see, *e.g.,* FIGs. 8A-C and **9**).

Pain arising from somatic or nerve injury/pathologies typically arises by activation of populations of primary afferent neurons which are characterized by activation thresholds associated with tissue injury and by sensitivity to products released by local tissue injury and inflammation. These afferents terminate in the spinal dorsal horn, where this input is encoded and transmitted by long ascending tracts to the brain, where it is processed into the pain experience. The cell body of a primary afferent lies in its dorsal root ganglion (DRG). These neuronal cell bodies, synthesize the voltage gated sodium channels that serve to initiate and propagate the action potential. While local anesthetics can yield a dense anesthesia, previous work has in fact shown that nonspecific sodium channel blockers such as lidocaine delivered systemically at subanesthetic concentrations were able to have selective effects upon hyperpathia in animal models and humans.

It is now known that there are nine voltage-gated sodium channel subtypes along with numerous splice variants. Of note, three of these isotypes: Naᵥ1.7, Naᵥ1.8, and Naᵥ1.9 have been found to be principally expressed in primary afferent nociceptors. The relevance of these isotypes to human pain has been suggested by the observation that a loss-of-function mutation in Naᵥ1.7 (SCN9A) leads to congenital insensitivity to pain (CIP), a rare genetic disorder. Conversely, gain of function mutations yield anomalous hyperpathic states. Based on these observations, the Naᵥ1.7 channel has been considered an attractive target for addressing pathologic pain states and for developing chronic pain therapies. Efforts to develop selective small molecule inhibitors have, however, been hampered due to the sequence similarity between Naᵥ subtypes. Many small-molecule drugs targeting Naᵥ1.7 have accordingly failed due to side effects caused by lack of targeting specificity or their bioavailability by the systemic route. Additionally, antibodies have faced a similar situation, since there is a tradeoff between selectivity and potency due to the binding of a specific (open or close) conformation of the channel, with binding not always translating into successful channel inhibition. Further, it is not clear that such antibodies can gain access to the appropriate Naᵥ1.7 channels and yield a reliable block of their function. Consequently, in spite of preclinical studies demonstrating that decreased Naᵥ1.7 activity leads to a reduction in inflammatory and neuropathic pain, no molecule targeting this gene product has reached the final phase of clinical trials.

This disclosure provides an alternative approach to the foregoing by epigenetically modulating the expression of Naᵥ1.7 using two genome engineering tools, clustered regularly interspaced short palindromic repeats (CRISPR)-Cas9 (CRISPR-Cas9) and zinc-finger proteins (ZFP), such that one could engineer highly specific, long-lasting and reversible treatments for pain.

Through its ability to precisely target disease-causing DNA mutations, the CRISPR-Cas9 system has emerged as a potent tool for genome manipulation, and has shown therapeutic efficacy in multiple animal models of human diseases. However, permanent genome editing, leading to permanent alteration of pain perception, may not be desirable. For example, pain can be a discomforting sensory and emotional experience, but it plays a critical role alerting of tissue damage. Permanent ablation could thus have detrimental consequences. For these reasons, a catalytically inactivated "dead" Cas9 (dCas9, also known as CRISPRi) has been employed herein, which does not cleave DNA but maintains its ability to bind to the genome via a guide-RNA (gRNA), and further fused the inactivated Cas9 to a repressor domain (Krüppel-associated box, KRAB) to enable non-permanent gene repression of Naᵥ1.7.

As shown herein, by addition of a KRAB epigenetic repressor motif to dCas9, gene repression can be enhanced with a high level of specificity both *in vitro* and *in vivo.* This transcriptional modulation system takes advantage of the high specificity of CRISPR-Cas9 while simultaneously increasing the safety profile, as no permanent modification of the genome is performed.

As a second approach for *in situ* epigenome repression of Naᵥ1.7, zinc-finger-KRAB proteins (ZFP-KRAB) have been utilized herein comprising a DNA-binding domain made up of Cys₂His₂ zinc fingers fused to a KRAB repressor. ZFP constitutes the largest individual family of transcriptional modulators encoded by the genomes of higher organisms, and with prevalent synthetic versions engineered on human protein chasses present a potentially low immunogenicity *in vivo* targeting approach. The disclosure further provides for specific anatomic targeting of the gene regulation by delivering both epigenetic tools (described herein) in an adeno-associated virus (AAV) construct (*e.g*., AAV1-9, rh.8, rh.10, rh.39 and rh.43) into the spinal intrathecal space. Of note, many AAVs have been shown to produce a robust transduction of the dorsal root ganglion. This approach has several advantages as it permits the use of minimal viral loads and reduces the possibility of systemic immunogenicity.

The terms "CRISPR system," "Cas system" or "CRISPR/Cas system" refer to a set of molecules comprising an RNA-guided nuclease or other effector molecule and a gRNA molecule that together direct and effect modification of nucleic acid at a target sequence by the RNA-guided nuclease or other effector molecule. In one embodiment, a CRISPR system comprises a gRNA and a Cas protein, *e.g.,* a Cas9 protein. Such systems comprising a Cas9 or modified Cas9 molecule are referred to herein as "Cas9 systems" or "CRISPR/Cas9 systems." In one example, the gRNA molecule and Cas molecule may be complexed, to form a ribonuclear protein (RNP) complex.

The terms "Cas9" or "Cas9 molecule" refer to an enzyme from bacterial Type II CRISPR/Cas system responsible for DNA cleavage. Cas9 also includes wild-type protein as well as functional and non-functional mutants thereof. In embodiments, the Cas9 is a Cas9 of *S. pyogenes* or *C*. *jejuni.* In a further embodiment, the Cas9 is a modified or "dead" Cas9 (dCas9). In still another embodiment, the Cas9 is a dead Cas9 that has been further truncated to limit its size. The disclosure contemplates the use of Cas9 nuclease-null orthologs from *Staphylococcus aureus, Streptococcus pyogenes, Streptococcus thermophilis, Treponema denticola, Neisseria meningitidis, Campylobacter jejuni, etc.*

In some embodiments, a Cas protein is modified (*e.g.* genetically engineered) to lack nuclease activity. For example, dead Cas9 (dCas9) protein binds to a target locus but does not cleave the nucleic acid at the locus. In some embodiments, a dCas9 protein comprises the sequence of SEQ ID NO:2 (the nucleic acid sequence is provided in SEQ ID NO:1). In other embodiments, the dCas9 comprises a sequence that is at least 70%, 80%, 85%, 87%, 90%, 92%, 95%, 98% or 99% identical to SEQ ID NO:2 and is capable of binding to a target sequence yet lacks nuclease activity.

In some embodiments, a catalytically dead Cas9 protein (*e.g.,* dead Cas9, "dCas9") is fused (*e.g.,* covalently bound) to a transcriptional regulator domain to modulate (*e.g.,* inhibit) expression of a target gene (*e.g.,* Nav1.7). In some embodiments, dCas9 comprises a sequence that is 70%-100% identical to SEQ ID NO:2. Without wishing to be bound by any particular theory, dCas9 (or another catalytically dead Cas protein) mediates transcriptional repression, in some embodiments, by sterically hindering the binding of transcriptional machinery (*e.g.,* a RNA polymerase complex) to a target sequence.

In some embodiments, the disclosure provides a split Cas9 system, wherein N- and C-domains are separated into different vectors and co-expressed with an N- and C-domain on an intein molecule. For example, two or more portions or segments of a Cas9 are provided to a cell, such as by being expressed from corresponding nucleic acids introduced into the cell. The two or more portions are combined within the cell to form the Cas9 which has an ability to colocalize with guide RNA at a target nucleic acid. It is to be understood that the Cas9 may have one or more modifications from a full length Cas9 known to those of skill in the art (*e.g*., dCas9), yet still retain or have the capability of colocalizing with guide RNA at a target nucleic acid. Accordingly, the two or more portions or segments, when joined together, need only produce or result in a Cas9 which has an ability to colocalize with guide RNA at a target nucleic acid. In one embodiment, the first nucleic acid encodes a first portion of the Cas9 protein having a first split-intein and wherein the second nucleic acid encodes a second portion of the Cas9 protein having a second split-intein complementary to the first split-intein and wherein the first portion of the Cas9 protein and the second portion of the Cas9 protein are joined together to form the Cas9 protein. In one embodiment, a C-Intein-dCCas9 comprises the sequence of SEQ ID NO:8 or a sequence that is 70-99% identical to SEQ ID NO:8 and the dNCas9-N-Intein comprises the sequence of SEQ ID NO:9 or a sequence that is 70-99% identical to SEQ ID NO:9.

In some embodiments, a Cas protein (*e.g*., dCas9) is fused to a transcriptional regulator domain. As used herein a "transcriptional regulator domain" is a protein domain that catalyzes structural or chemical changes in a chromatin molecule that results in altered transcriptional activity (*e.g*., transcriptional activation or transcriptional repression). In some embodiments, the transcriptional regulator domain is a transcriptional repressor domain. In some embodiments, the repressive domain comprises a Kruppel associated box domain (KRAB domain). Non-limiting examples of KRAB domains include KOX1 KRAB domain, KOX8 KRAB domain, ZNF43 KRAB domain, and ZNF184 KRAB domain. In some embodiments, the KRAB domain is a KOX1 KRAB domain. Further non-limiting examples of repressive domains include Chromo Shadow (CS) domain (*e.g.,* CS domain of HP1α) and WRPW domain (*e.g*., WRPW domain of Hes1). In a particular embodiment, the KRAB domain comprises the nucleic acid sequence of SEQ ID NO:6 and the polypeptide sequence of SEQ ID NO:7 or sequences that are at least 70%, 80%, 85%, 87%, 90%, 92%, 95%, 98%, or 99% identical thereto).

In some embodiments, the dCas9 comprises one or more of a transcriptional repressor. For example, in some embodiments, the general architecture of exemplary dCas9 fusion proteins with a transcriptional repressor domain comprises the structure: [NH₂]-[NLS]-[dCas9 or Cas9]-[(transcriptional repressor)ₙ]-[COOH], [NH₂]-[NLS]-[(transcriptional repressor) ₙ]-[dCas9 or Cas9]-[COOH], [NH₂]-[dCas9 or Cas9]-[(transcriptional repressor)ₙ]-[COOH], or [NH₂]-[(transcriptional repressor)ₙ]-[dCas9 or Cas9]-[COOH]; wherein NLS is a nuclear localization signal, NH₂ is the N-terminus of the fusion protein, and COOH is the C-terminus of the fusion protein. In some embodiments, the fusion proteins comprises one or more repeats of the transcriptional repressor, for example wherein n=1-10 (*e.g.*, n is 1, 2, 3, 4, 5, 6, 7, 8, 9, or 10). In some embodiments, n=1-20. In some embodiments, a linker is inserted between the dCas9 and the transcriptional repressor domain. In some embodiments, a linker is inserted between the nuclear localization signal (NLS) and the transcriptional repressor and/or dCas9 domain. In some embodiments, the NLS is located C-terminal of the transcriptional repressor and/or the dCas9 domain. In some embodiments, the NLS is located between the transcriptional repressor domain and the dCas9 domain. Additional features, such as sequence tags, may also be present. In some embodiments, the transcriptional repressor is selected from the group consisting of the KRAB (Kruppel associated box) domain of Kox1, SID (mSin3 interaction domain), the CS (Chromo Shadow) domain of HP1α, the WRPW domain of Hesl, MBD2, MBD3, DNMT family (DNMT1, DNMT3A, DNMT3B, DNMT2A), Rb, Mecp2, Fog1,ROM2, AtHD2A, and LSD1. These and other repressor domains are known in the art, and in some embodiments correspond to those described in Urrutia, KRAB-containing zinc-finger repressor proteins. Genome Biol. 2003; 4(10):231; Gilbert et al. CRISPR-mediated modular RNA-guided regulation of transcription in eukaryotes. Cell. 2013; 154, 442-451; Konermann et al., Optical control of mammalian endogenous transcription and epigenetic states. Nature. 2013; 500, 472-476; and published U.S. patent application Ser. No. 14/105,017, published as U.S. 2014/0186958 A1, the entire contents of which are incorporated herein by reference. In some embodiments, the transcription repressor domain comprises one or more repeats (*e.g.,* 2, 3, 4, 5, 6, 7, 8, 9, or 10 repeats) of a KRAB domain. In some embodiments, the KRAB domain comprises an amino acid sequence selected from the group consisting of SEQ ID NO:7. In some embodiments, the transcriptional repressor domains comprises one or more repeats of a SID protein. In some embodiments, the repressor domain comprises 2, 3, 4, 5, 6, 7, 8, 9, or 10 repeats of a SID protein. In some embodiments, the repressor domain comprises four repeats of SID.

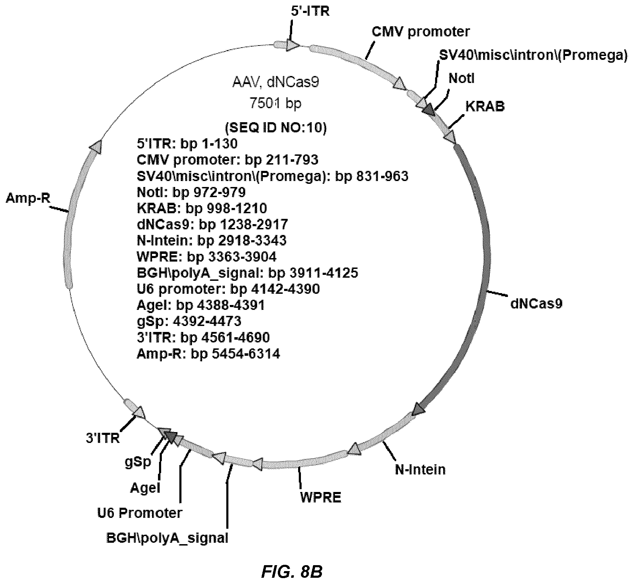

In some embodiments, the transcription regulator is present in one or both construct(s) of a split-Cas9 system. For example, a KRAB domain can be present in either or both of SEQ ID NOs: 3 and/or 4. For example, when the KRAB sequence is present in SEQ ID NO:3 the resulting construct is provided in FIG. 8B and SEQ ID NO:10.

The terms "guide RNA," "guide RNA molecule," "gRNA molecule" or "gRNA" are used interchangeably, and refer to a set of nucleic acid molecules that promote a RNA-guided nuclease or other effector molecule (typically in complex with the gRNA molecule) to a target a specific sequence. Techniques of designing gRNAs and donor therapeutic polynucleotides for target specificity are well known in the art. For example, Doench, J., et al. Nature biotechnology 2014; 32(12):1262-7, Mohr, S. et al. (2016) FEBS Journal 283: 3232-38, and Graham, D., et al. Genome Biol. 2015; 16: 260. gRNA comprises or alternatively consists essentially of, or yet further consists of a fusion polynucleotide comprising CRISPR RNA (crRNA) and trans-activating CRIPSPR RNA (tracrRNA); or a polynucleotide comprising CRISPR RNA (crRNA) and trans-activating CRIPSPR RNA (tracrRNA). In some aspects, a gRNA is synthetic (Kelley, M. et al. (2016) J of Biotechnology 233 (2016) 74-83). In some embodiments, the targeting is accomplished through hybridization of a portion of the gRNA to DNA (*e.g.*, through the gRNA targeting domain), and by binding of a portion of the gRNA molecule to the RNA-guided nuclease or other effector molecule. In embodiments, a gRNA molecule consists of a single contiguous polynucleotide molecule, referred to herein as a "single guide RNA" or "sgRNA" and the like. In other embodiments, a gRNA molecule consists of a plurality, usually two, polynucleotide molecules, which are themselves capable of association, usually through hybridization, referred to herein as a "dual guide RNA" or "dgRNA," and the like. gRNA molecules generally include a targeting domain and a tracr. In embodiments the targeting domain and tracr are disposed on a single polynucleotide. In other embodiments, the targeting domain and tracr are disposed on separate polynucleotides.

The term "targeting domain" as the term is used in connection with a gRNA, is the portion of the gRNA molecule that recognizes, *e.g.,* is complementary to, a target sequence, *e.g.,* a target sequence within the nucleic acid of a cell, *e.g.,* within a gene.

The term "target sequence" refers to a sequence of nucleic acids complimentary, for example fully complementary, to a gRNA targeting domain. In embodiments, the target sequence is disposed on genomic DNA. In an embodiment the target sequence is adjacent to (either on the same strand or on the complementary strand of DNA) a protospacer adjacent motif (PAM) sequence recognized by a protein having nuclease or other effector activity, *e.g.,* a PAM sequence recognized by Cas9. In embodiments, the target sequence is a target sequence within a gene or locus that affects expression of a Nav1.7 gene, *e.g.,* that affects expression of voltage gated sodium channel 1.7.

The disclosure provides gRNA targeting sequences in Table 1. It will be recognized that the gRNA targeting sequences in Table 1 can vary by substitution of about 1-5 base pairs (*e.g*., 1, 2, 3, 4, or 5 base pairs) so long as the targeting sequence is able to hybridize to the target sequence in the SCN9A gene in the genome.

**Table 1:**

| Targeting sequence (gRNA) |
|---|
| ACAGTGGGCAGGATTGAAA (SEQ ID NO:11) |
| GCAGGTGCACTCACCGGGT (SEQ ID NO:12) |
| GAGCTCAGGGAGCATCGAGG (SEQ ID NO:13) |
| AGAGTCGCAATTGGAGCGC (SEQ ID NO:14) |
| CCAGACCAGCCTGCACAGT (SEQ ID NO:15) |
| GAGCGCAGGCTAGGCCTGCA(SEQ ID NO:16) |
| CTAGGAGTCCGGGATACCC (SEQ ID NO:17) |
| GAATCCGCAGGTGCACTCAC(SEQ ID NO:18) |
| GACCAGCCTGCACAGTGGGC(SEQ ID NO:19) |
| GCGACGCGGTTGGCAGCCGA(SEQ ID NO:20) |
| GGTCGCCAGCGCTCCAGCGG(SEQ ID NO:21) |
| GCTTTCCAATTCCGCCAGCT(SEQ ID NO:22) |
| CAATTCCGCCAGCTCGGCTG(SEQ ID NO:23) |
| CCCAGCCTCAGCCGAGCTGG(SEQ ID NO:24) |
| CCGCCAGCTCGGCTGAGGCT(SEQ ID NO:25) |
| GGAAAGCCGACAGCCGCCGC(SEQ ID NO:26) |
| AGCGCTCCAGCGGCGGCTGT(SEQ ID NO:27) |
| GGCGGTCGCCAGCGCTCCAG(SEQ ID NO:28) |
| CTCAGCCGAGCTGGCGGAAT(SEQ ID NO:29) |
| TAGCCCAGCCTCAGCCGAGC(SEQ ID NO:30) |
| GGCGGTCGCCAGCGCTCCAG(SEQ ID NO:31) |
| GCCACCTGGAAAGAAGAGAG(SEQ ID NO:32) |
| GGTCGCCAGCGCTCCAGCGG(SEQ ID NO:33) |
| GCCAGCAATGGGAGGAAGAA(SEQ ID NO:34) |
| GTTCCAGGTGGCGTAATACA(SEQ ID NO:35) |
| GGCGGGGCTGCTACCTCCAC(SEQ ID NO:36) |
| GGGCGCAGTCTGCTTGCAGG(SEQ ID NO:37) |
| GGCGCTCCAGCGGCGGCTGT(SEQ ID NO:38) |
| GACCGGGTGGTTCCAGCAAT(SEQ ID NO:39) |
| GGGGTGGTTCCAGCAATGGG(SEQ ID NO:40) |
| GGGCGCAGTCTGCTTGCAGG(SEQ ID NO:41) |
| TGGGTGCCAGTGGCTGCTAG(SEQ ID NO:42) |
| TCTGGGCTCCTGTTGCTCAG(SEQ ID NO:43) |
| GCAGCCCTGAGAGAGCGCCG(SEQ ID NO:44) |
| GAGCACGGGCGAAAGACCGA(SEQ ID NO:45) |
| ATAGACACAGGTGGGTGTGG(SEQ ID NO:46) |
| ATGTGAAAATAGACACAGGT(SEQ ID NO:47) |
| GATGTGAAAATAGACACAGG(SEQ ID NO:48) |
| CGAGATGTGAAAATAGACAC(SEQ ID NO:49) |
| GCCACCTGGAAAGAAGAGAG(SEQ ID NO:50) |
| AGGGGAGAAGCTTGACCGGG(SEQ ID NO:51) |
| CGGGTGGTTCCAGCAATGGG(SEQ ID NO:52) |
| ATAGCTGGGCAGCTCCTGTG(SEQ ID NO:53) |
| CCACAGAGTCAAAACCGCAC(SEQ ID NO:54) |
| GCTGCCAGGTTCTGAAACTG(SEQ ID NO:55) |
| CAGGTTCTGAAACTGTGGAA(SEQ ID NO:56) |
| AAAGGAAGGGTAGCAATGCC(SEQ ID NO:57) |
| GGAAGGGTAGCAATGCCTGG(SEQ ID NO:58) |
| ATAAAAGACAGTAAACCACC(SEQ ID NO:59) |
| TAGATGGACTTCAATTCAAG(SEQ ID NO:60) |
| GCTTAGCAGATACAACCTGT(SEQ ID NO:61) |
| CTTAGCAGATACAACCTGTG(SEQ ID NO:62) |
| AATTTACATGAGAAACTTAG(SEQ ID NO:63) |
| ATTTACATGAGAAACTTAGG(SEQ ID NO:64) |
| TTTACATGAGAAACTTAGGG(SEQ ID NO:65) |
| TCATGAAAATTTGCGACACA(SEQ ID NO:66) |
| TGATTATATGCAGGCCCTAG(SEQ ID NO:67) |
| TAATCATGGGAGCCCTTCTG(SEQ ID NO:68) |
| ATAGAAGCATTACCACAGAA(SEQ ID NO:69) |
| TAATCAACCCACTTTCTCTG(SEQ ID NO:70) |
| AACCCACTTTCTCTGTGGCA(SEQ ID NO:71) |
| gccgtgtagatacagaaaag(SEQ ID NO:72) |
| gtatagagaatgaattgcag(SEQ ID NO:73) |
| tgtatagagaatgaattgca(SEQ ID NO:74) |
| ATTTaaaaaaaaaaaaaaaG(SEQ ID NO:75) |
| AGAGAGTAAACCATATGCTG(SEQ ID NO:76) |
| gaagagaataggttctggtg(SEQ ID NO:77) |
| atgtgttttagccacgacct(SEQ ID NO:78) |
| TCCAACATCAAGACCAACAC(SEQ ID NO:79) |
| TTCCAACATCAAGACCAACA(SEQ ID NO:80) |
| TTTGCATACCAAATACTCCA(SEQ ID NO:81) |
| TTGCATACCAAATACTCCAA(SEQ ID NO:82) |
| gcctggcatcaagtagtagg(SEQ ID NO:83) |
| ATCATGGTATGATATTGAGG(SEQ ID NO:84) |
| AGAAATGTAGTCAGATGAGG(SEQ ID NO:85) |
| CCATAAGTTAGGTTTCCACA(SEQ ID NO:86) |
| AAACATCAATTTAGACCGTG(SEQ ID NO:87) |
| TCTCTAAGGAAGGTTCAGAG(SEQ ID NO:88) |
| GAAGGTTCAGAGAGGCAATG(SEQ ID NO:89) |
| ATAGTCTGCAAAAATAAAGG(SEQ ID NO:90) |
| AATTATTTACCAAAAATCTG(SEQ ID NO:91) |
| ATTGTGGATGTTGTATTGGA(SEQ ID NO:92) |
| AGGAATGAAACCCTTCTGGG(SEQ ID NO:93) |
| TGCCAGGCCATGATAAAGTG(SEQ ID NO:94) |
| ACAGGAGCCCAGAGAAAAAG(SEQ ID NO:95) |
| GGAGCCCAGAGAAAAAGAAG(SEQ ID NO:96) |
| ACAGAGTCAAAACCGCACAG(SEQ ID NO:97) |
| GCGTAAACAGAAATAAAAGA(SEQ ID NO:98) |
| TCTGCGCTGAGAAATAGGGG(SEQ ID NO:99) |
| TTTGCTTCTGAAACTCAGCA(SEQ ID NO:100) |
| GTTGCTGTGCTGAGTTTCAG(SEQ ID NO:101) |
| CTACTTTTTTCCTTGCCACA(SEQ ID NO:102) |
| gctgaaatggagtaataagg(SEQ ID NO:103) |
| atagagaatgaattgcaggg(SEQ ID NO:104) |
| gaatagtgcctggcatcaag(SEQ ID NO:105) |
| GTAATGCATTCTTAGAAAGG(SEQ ID NO:106) |
| GTAGAGTTAGATTACCACTT(SEQ ID NO:107) |

In one embodiment, the disclosure provides a recombinant gene repressor complex comprising a nuclease inactivated Cas9 protein fused to a transcription repressor and wherein the nuclease inactivated Cas9 is associated with a guide RNA wherein the guide RNA has a sequence selected from SEQ ID Nos: 11-107, and sequences of any of SEQ ID Nos:11-107 having 1-5 (*e.g*., 1, 2, 3, 4, or 5) base pair substitutions, wherein the gRNA can bind to a target sequence in the SCN9A gene repress expression of the gene. In one embodiment, the nuclease inactivated Cas9 is in the form of a split Cas9. The inactivated Cas9 can be obtained and/or derived from any Cas9 protein. In another embodiment, the nuclease inactivated Cas9 is engineered from *C*. *jejuni.* In another embodiment, the nuclease inactivated Cas9 comprises SEQ ID NO:2 or a sequence that is at least 70%, at least 90%-99% identical thereto. In another or further embodiment, the transcription repressor is KRAB. In a further embodiment, the KRAB comprises a sequence as set forth in SEQ ID NO:7 or a sequence that is 70%-99% identical to SEQ ID NO:7 and which can repress gene transcription.

With reference to the constructs in FIG 8A-C, at least one of the gRNA sequences of Table 1 are cloned into the AgeI site just downstream of the U6 promoter.

As discussed further below, the recombinant gene repressor complex can be delivered using various viral vectors including lentiviral vectors and adenoviral vectors.

The disclosure also provides recombinant gene repressor complexes comprising zinc finger DNA binding proteins.

As used herein "polyA" refers to a polymer of adenosines. The polyA sequences can be obtained from, for example, SV40 poly(A), bovine growth hormone poly(A) (bGHpA), rabbit β-globin poly(A) and the like.

"Regulatory elements" can be used in the methods and compositions to improve expression from a viral vector. For example, Woodchuck Hepatitis Virus Regulatory Element (WPRE) can be used to enhance expression of a dCas9 construct from a viral vector. The sequence of WPRE is known (see, *e.g.,* "WHP Posttranscriptional Response Element" in WIKIPEDIA;
[https://]en.wikipedia.org/wiki/WHP_Posttranscriptional_Response_Ele ment).

Referring to the constructs presented in Figure 8A-C and 9, one of skill in the art will notice that the structures are modular and thus different "polyA" sequences as provided herein can be cloned into a vector of the disclosure; different promoters can be substituted into the vector in place of the "CMV" in Figure 8A-C and 9 (*e.g*., the CMV promoter can be replaced by another polII promoter such as an RSV promoter); different polIII promoters can be substituted for the U6 promoter in Figure 8A-C *etc*.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein, ZF or ZFP. The individual DNA binding domains are typically referred to as "fingers." A ZFP has at least one finger, typically two, three, four, five, six or more fingers. Each finger binds from two to four base pairs of DNA, typically three or four base pairs of DNA. A ZFP binds to a nucleic acid sequence called a target site or target segment. Each finger typically comprises an approximately 30 amino acid, zinc-chelating, DNA-binding subdomain. An exemplary motif characterizing one class of these proteins (C₂H₂ class) is - Cys-(X)₂₋₄-Cys-(X)₁₂-His-(X)₃₋₅-His (where X is any amino acid) (SEQ ID NO:143). Additional classes of zinc finger proteins are known and are useful in the practice of the methods, and in the manufacture and use of the compositions disclosed herein (see, *e.g.,* Rhodes et al. (1993) Scientific American 268:56-65 and US Patent Application Publication No. 2003/0108880). Studies have demonstrated that a single zinc finger of this class consists of an alpha helix containing the two invariant histidine residues coordinated with zinc along with the two cysteine residues of a single beta turn (see, *e.g.,* Berg & Shi, Science 271:1081-1085 (1996)). A single target site for ZFP typically has about four to about ten base pairs. Typically, a two-fingered ZFP recognizes a four to seven base pair target site, a three-fingered ZFP recognizes a six to ten base pair target site, a four-finger ZFP recognizes a twelve to fourteen base pair target site and a six-fingered ZFP recognizes an eighteen to twenty base pair target site, which can comprise two adjacent nine to ten base pair target sites or three adjacent six to seven base pair target sites.

For those embodiments comprising an engineered zinc finger binding domain, the zinc finger domain is engineered to bind a specific target site. The binding domain contains a plurality of zinc fingers (*e.g*., 2, 3, 4, 5, 6 or more zinc fingers). In general, an individual zinc finger binds a subsite of 3-4 nucleotides. The subsites can be contiguous in a target site (and are in some cases overlapping); alternatively a subsite can be separated from an adjacent subsite by gaps of one, two three or more nucleotides. Binding to subsites separated by a gap of one or more nucleotides is facilitated by the use of non-canonical, longer linker sequences between adjacent zinc fingers.

The disclosure provides zinc finger targets as set forth in Table 2. Table 2 provide both murine and human target sites in the SCN9A gene sequence.

**Table 2:**

| Name | | Target sequence |
|---|---|---|
| mScn10a_1 | (ZF-1) | tgAGTGACGGACGGGTGAGGtttccgtc (SEQ ID NO:108) |
| mScn10a_2 | (ZF-2) | ttCGTGGAGGAGCCCCGGACaagtnnnn(SEQ ID NO:109) |
| mScn10a_3 | (ZF-3) | atGGTGCTCCAGAAAGTACActctgaat(SEQ ID NO:110) |
| mScn10a_4 | (ZF-4) | tgAGTGACGGACGGGTGAGGtttccgtc(SEQ ID NO:111) |
| hSCN9A_1 | | AGTCTGCTTGCAGGCGGT(SEQ ID NO:112) |
| hSCN9A_2 | | CCAGCGGCGGCTGTCGGC(SEQ ID NO:113) |
| hSCN9A_3 | | GCCTGGGTGCCAGTGGCT(SEQ ID NO:114) |
| hSCN9A_4 | | TGGCTGCTAGCGGCAGGC(SEQ ID NO:115) |
| hSCN9A_5 | | GCGTCCCCTGAGCAACAG(SEQ ID NO:116) |
| hSCN9A_6 | | AAGGAGAGGCCCGCGCCC(SEQ ID NO:117) |
| hSCN9A_7 | | GCAGGTGCACTGGGTGGG(SEQ ID NO:118) |
| hSCN9A_8 | | GCGCCCGTGGAGGTAGCA(SEQ ID NO:119) |
| hSCN9A_9 | | TGCCAGGGCGCGCCCGTG(SEQ ID NO:120) |
| hSCN9A_10 | | ACAGCCGCCGCTGGAGCG(SEQ ID NO:121) |
| hSCN9A_11 | | CCAGGAGAGGGCGCGGGC(SEQ ID NO:122) |

Using the targeting sequences above, one of skill in the art can design zinc-finger proteins to bind to these sequences. For example, using methods known in the art, zinc-finger repressor constructs were designed to bind to SEQ ID Nos:108, 109, 110 or 111. The zinc-finger repressor constructs comprise 6 zinc fingers as set forth in Table 3 below, wherein the targeting amino acids of the zinc-finger comprise from 6-12 amino acids.

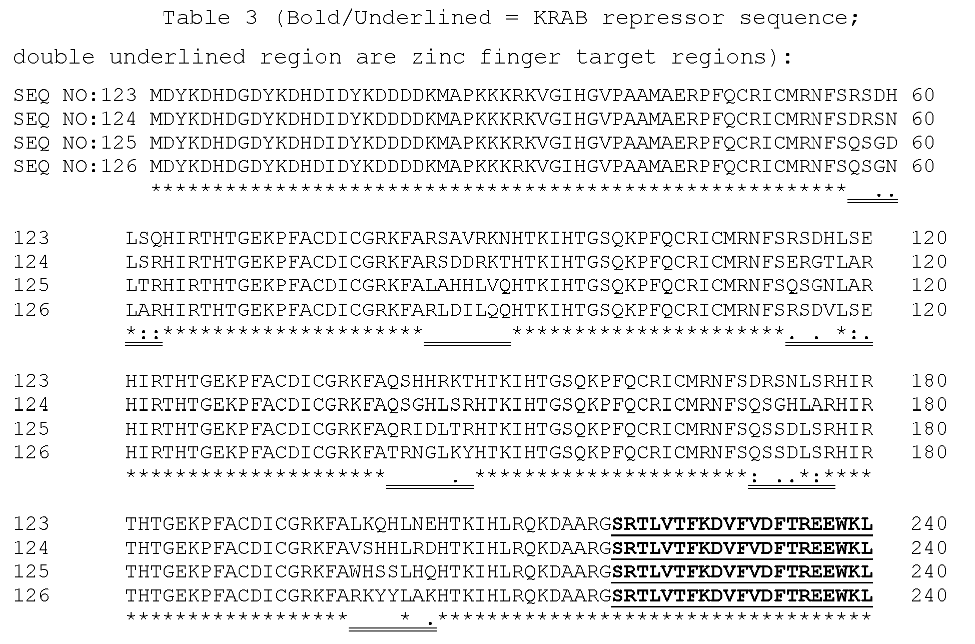

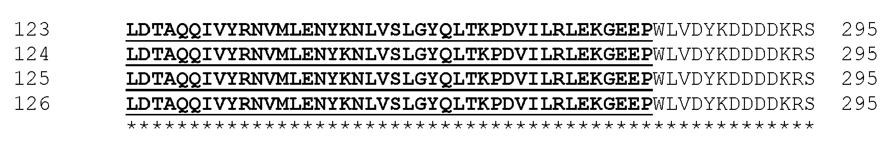

Human Nav1.7 expression is regulated by ZFPs through binding to a target site with nucleic acid sequences set forth in Table 2 (*e.g.,* SEQ ID Nos: 108-122 or a subsequence thereof. Murine Nav1.7 expression is regulated by the ZFPs through binding to a target site having a sequence of SEQ ID NO:108, 109, 110, or 111. Species variants of NAV1.7 (such as murine SCN10A) can be regulated at the corresponding site (*i.e.*, site having greatest sequence identity) to SEQ ID NO:108, 109, 110 or 111 in that species. Nucleotides comprising subsites to which individual zinc fingers primarily contact are shown in uppercase. Nucleotides between subsites are shown in lowercase.

Exemplary ZFP-KRAB sequences that can repress Nav1.7 expression in the mouse homolog (SCN10A) are provided in SEQ ID NOs:123-126, wherein each sequence includes 6 finger domains and the KRAB sequence from amino acid 220 to 282.

Zinc finger or CRISPR/Cas proteins as described herein may be delivered using vectors containing sequences encoding one or more of the zinc finger or CRISPR/Cas protein(s). Any vector systems may be used including, but not limited to, plasmid vectors, retroviral vectors, lentiviral vectors, adenovirus vectors, poxvirus vectors; herpesvirus vectors and adeno-associated virus vectors, etc. See, also, U.S. Pat. Nos. 6,534,261; 6,607,882; 6,824,978; 6,933,113; 6,979,539; 7,013,219; and 7,163,824, incorporated by reference herein in their entireties. Furthermore, it will be apparent that any of these vectors may comprise one or more zinc finger protein-encoding sequences. Thus, when one or more ZFPs or CRISPR/Cas proteins are introduced into the cell, the sequences encoding the ZFPs or CRISPR/Cas proteins may be carried on the same vector or on different vectors. When multiple vectors are used, each vector may comprise a sequence encoding one or multiple ZFPs or CRISPR/Cas systems.

Conventional viral and non-viral based gene transfer methods can be used to introduce nucleic acids encoding engineered ZFPs or CRISPR/Cas systems in cells (*e.g.,* mammalian cells) and target tissues. Such methods can also be used to administer nucleic acids encoding ZFPs or a CRISPR/Cas system to cells *in vitro.* In certain embodiments, nucleic acids encoding the ZFPs or CRISPR/Cas system are administered for *in vivo* or *ex vivo* gene therapy uses. Non-viral vector delivery systems include DNA plasmids, naked nucleic acid, and nucleic acid complexed with a delivery vehicle such as a liposome or poloxamer. Viral vector delivery systems include DNA and RNA viruses, which have either episomal or integrated genomes after delivery to the cell. Gene therapy procedures are known, see Anderson (1992) Science 256:808-813; Nabel and Felgner (1993) TIBTECH 11:211-217; Mitani and Caskey (1993) TIBTECH 11:162-166; Dillon (1993) TIBTECH 11:167-175; Miller (1992) Nature 357:455-460; Van Brunt (1988) Biotechnology 6(10):1149-1154; Vigne (1995) Restorative Neurology and Neuroscience 8:35-36; Kremer and Perricaudet (1995) British Medical Bulletin 51(1):31-44; Haddada, et al., in Current Topics in Microbiology and Immunology Doerfler and Bohm (eds.) (1995); and Yu, et al. (1994) Gene Therapy 1:13-26.

Methods of non-viral delivery of nucleic acids include electroporation, lipofection, microinjection, biolistics, virosomes, liposomes, immunoliposomes, exosomes, polycation or lipid:nucleic acid conjugates, naked DNA, naked RNA, artificial virions, and agent-enhanced uptake of DNA. Sonoporation using, *e.g.,* the Sonitron 2000 system (Rich-Mar) can also be used for delivery of nucleic acids. In one embodiment, one or more nucleic acids are delivered as mRNA. Also in some embodiments, capped mRNAs can be used to increase translational efficiency and/or mRNA stability.

Additional exemplary nucleic acid delivery systems include those provided by Amaxa Biosystems (Cologne, Germany), Maxcyte, Inc. (Rockville, Md.), BTX Molecular Delivery Systems (Holliston, Mass.) and Copernicus Therapeutics Inc, (see for example U.S. Pat. No. 6,008,336). Lipofection is described in *e.g.,* U.S. Pat. Nos. 5,049,386; 4,946,787; and 4,897,355) and lipofection reagents are sold commercially (*e.g.,* Transfectam^{™} and Lipofectin^{™} and Lipofectamine^{™} RNAiMAX). Cationic and neutral lipids that are suitable for efficient receptor-recognition lipofection of polynucleotides include those of Felgner, International Patent Publication Nos. WO 91/17424 and WO 91/16024. Delivery can be to cells (*ex vivo* administration) or target tissues (in *vivo* administration).

The preparation of lipid:nucleic acid complexes, including targeted liposomes such as immunolipid complexes, is well known to one of skill in the art (see, *e.g.,* Crystal (1995) Science 270:404-410 (1995); Blaese, et al. (1995) Cancer Gene Ther. 2:291-297; Behr, et al. (1994) Bioconjugate Chem. 5:382-389; Remy, et al. (1994) Bioconjugate Chem. 5:647-654; Gao, et al. (1995) Gene Therapy 2:710-722; Ahmad, et al. (1992) Cancer Res. 52:4817-4820; U.S. Pat. Nos. 4,186,183; 4,217,344; 4,235,871; 4,261,975; 4,485,054; 4,501,728; 4,774,085; 4,837,028; and 4,946,787).

Additional methods of delivery include the use of packaging the nucleic acids to be delivered into EnGenelC delivery vehicles (EDVs). These EDVs are specifically delivered to target tissues using bispecific antibodies where one arm of the antibody has specificity for the target tissue and the other has specificity for the EDV. The antibody brings the EDVs to the target cell surface and then the EDV is brought into the cell by endocytosis. Once in the cell, the contents are released (see MacDiarmid, et al. (2009) Nature Biotechnology 27(7):643).

The use of RNA or DNA viral based systems for the delivery of nucleic acids encoding engineered ZFPs or CRISPR/Cas systems take advantage of highly evolved processes for targeting a virus to specific cells in the body and trafficking the viral payload to the nucleus. Viral vectors can be administered directly to patients (*in vivo*) or they can be used to treat cells *in vitro* and the modified cells are administered to patients (*ex vivo*)*.* Conventional viral based systems for the delivery of ZFPs or CRISPR/Cas systems include, but are not limited to, retroviral, lentivirus, adenoviral, adeno-associated, vaccinia and herpes simplex virus vectors for gene transfer.

Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon mouse leukemia virus (MuLV), gibbon ape leukemia virus (GaLV), Simian Immunodeficiency virus (SIV), human immunodeficiency virus (HIV), and combinations thereof (see, *e.g.,* Buchscher, et al. (1992) J. Virol. 66:2731-2739; Johann, et al. (1992) J. Virol. 66:1635-1640; Sommerfelt, et al. (1990) Virol. 176:58-59; Wilson, et al. (1989) J. Virol. 63:2374-2378; Miller, et al. (1991) J. Virol. 65:2220-2224 (1991); PCT/US94/05700).

In applications in which transient expression is desired, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, *e.g.,* in the *in vitro* production of nucleic acids and peptides, and for *in vivo* and *ex vivo* gene therapy procedures (see, *e.g.,* West, et al. (1987) Virology 160:38-47; U.S. Pat. No. 4,797,368; International Patent Publication No. WO 93/24641; Kotin (1994) Human Gene Therapy 5:793-801; Muzyczka (1994) J. Clin. Invest. 94:1351. Construction of recombinant AAV vectors are described in a number of publications, including U.S. Pat. No. 5,173,414; Tratschin, et al. (1985) Mol. Cell. Biol. 5:3251-3260; Tratschin, et al. (1984) Mol. Cell. Biol. 4:2072-2081; Hermonat and Muzyczka (1984) PNAS 81:6466-6470; and Samulski, et al. (1989) J. Virol. 63:3822-3828.

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar, et al. (1995) Blood 85:3048-305; Kohn, et al. (1995) Nat. Med. 1:1017-102; Malech, et al. (1997) PNAS 94(22):12133-12138). PA317/pLASN was the first therapeutic vector used in a gene therapy trial. (Blaese, et al. (1995) Science 270:475-480). Transduction efficiencies of 50% or greater have been observed for MFG-S packaged vectors. (Ellem, et al. (1997) Immunol Immunother 44(1):10-20; Dranoff, et al. (1997) Hum. Gene Ther. 1:111-112.

Recombinant adeno-associated virus vectors (rAAV) are a promising alternative gene delivery systems based on the defective and nonpathogenic parvovirus adeno-associated type 2 virus. All vectors are derived from a plasmid that retains only the AAV 145 bp inverted terminal repeats (ITRs) flanking the transgene expression cassette. Efficient gene transfer and stable transgene delivery due to integration into the genomes of the transduced cell are key features for this vector system. (Wagner, et al. (1998) Lancet 351(9117):1702-1703; Kearns, et al. (1996) Gene Ther. 9:748-55). Other AAV serotypes, including AAV1, AAV3, AAV4, AAVS, AAV6, AAV8AAV 8.2, AAV9, and AAV rh10 and pseudotyped AAV such as AAV2/8, AAV2/5 and AAV2/6 can also be used in accordance with the present disclosure.

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in trans. Ad vectors can transduce multiple types of tissues *in vivo,* including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman, et al. (1998) Hum. Gene Ther. 7:1083-1089). Additional examples of the use of adenovirus vectors for gene transfer in clinical trials include Rosenecker, et al. (1996) Infection 24(1):5-10; Sterman, et al. (1998) Hum. Gene Ther. 9(7):1083-1089; Welsh, et al. (1995) Hum. Gene Ther. 2:205-218; Alvarez, et al. (1997) Hum. Gene Ther. 5:597-613; Topf, et al. (1998) Gene Ther. 5:507-513; Sterman, et al. (1998) Hum. Gene Ther. 7:1083-1089.

Packaging cells are used to form virus particles that are capable of infecting a host cell. Such cells include 293 cells, which package adenovirus, and Ψ2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in *trans* by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, *e.g.,* heat treatment to which adenovirus is more sensitive than AAV.

In many gene therapy applications, it is desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han, et al. (1995) Proc. Natl. Acad. Sci. USA 92:9747-9751, reported that Moloney mouse leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (*e.g.,* FAB or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Gene therapy vectors can be delivered *in vivo* by administration to an individual patient, typically by systemic administration (*e.g*., intravenous, intraperitoneal, intramuscular, subdermal, intrathecal or intracranial infusion) or topical application, as described below. Alternatively, vectors can be delivered to cells *ex vivo,* such as cells explanted from an individual patient (*e.g*., lymphocytes, bone marrow aspirates, tissue biopsy) or universal donor hematopoietic stem cells, followed by reimplantation of the cells into a patient, usually after selection for cells which have incorporated the vector.

Vectors (*e.g*., retroviruses, adenoviruses, liposomes, etc.) containing therapeutic ZFP nucleic acids or CRISPRi can also be administered directly to an organism for transduction of cells *in vivo.* Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions available, as described below (see, *e.g.,* Remington's Pharmaceutical Sciences, 17th ed.

The Cas9 complexes and zinc finger-fusion constructs of the disclosure were used to inhibit pain perception in various animal models. Since pain perception is etiologically diverse and multifactorial, several rodent pain models were utilized to study pain signaling and pain behaviors. The studies described herein evaluated the effects of CRISPR-mediated knock down of Naᵥ1.7 using three mechanistically distinct models: (i) thermal sensitivity in control (normal) and unilateral inflammation-sensitized hind paw; (ii) a poly neuropathy induced by a chemotherapeutic yielding a bilateral hind paw tactile allodynia and, (iii) a spinally evoked bilateral hind paw tactile allodynia induced by spinal activation of purine receptors. Pain due to tissue injury and inflammation results from a release of factors that sensitize the peripheral terminal of the nociceptive afferent neuron. This phenotype can be studied through local application of carrageenan to the paw resulting in inflammation, swelling, increased expression of Naᵥ1.7 and a robust increase in thermal and mechanical sensitivity (hyperalgesia). Chemotherapy to treat cancer often leads to a polyneuropathy characterized by increased sensitivity to light touch (*e.g*. tactile allodynia) and cold. Paclitaxel is a commonly used chemotherapeutic that increases the expression of Naᵥ 1.7 in the nociceptive afferents and induces a robust allodynia in the animal models. Finally, ATP (adenosine triphosphate) by an action on a variety of purine receptors expressed on afferent terminals and second order neurons and non-neuronal cells has been broadly implicated in inflammatory, visceral and neuropathic pain states. Thus, intrathecal delivery of a stable ATP analogue (BzATP: 2',3'-O-(4-benzoylbenzoyl)-ATP) results in a long-lasting allodynia in mice.

Various KRAB-CRISPR-dCas9 and ZFP-KRAB constructs are provided herein and were studied for repressing the expression of Naᵥ1.7 (Neuro2a) in a mouse neuroblastoma cell line. These constructs can be packaged into a retroviral system for delivery. For example, the constructs tested having *in vitro* repression were packaged into AAV9 and injected intrathecally into adult C57BL/6J mice. After 21 days, paw inflammation was induced via injection of carrageenan. Thermal hyperalgesia was then evaluated. In the exemplary studies presented herein, *in vivo* repression of Naᵥ1.7 using the constructs of the disclosure provided a decrease in thermal hyperalgesia. The constructs were further tested in two neuropathic pain models: chemotherapy-induced (paclitaxel) and BzATP-induced neuropathic pain. The results in the paclitaxel-induced neuropathic pain model indicate that repression of Naᵥ1.7 using the constructs of the disclosure lead to reduced tactile and cold allodynia. In addition, KRAB-CRISPR-dCas9 injected mice showed reduced tactile allodynia after administration of the ATP analogue BzATP. As many pain states occur after chronic inflammation and nerve injury causing an enduring condition, typically requiring constant re-medication, the genetic approaches of the disclosure provide ongoing and controllable regulation of this aberrant processing and enduring pain. The *in situ* epigenetic approaches described herein represents a viable replacement for opioids and serve as a potential therapeutic approach for long lasting chronic pain.

In the experiments presented herein, the efficacy of the repression of Naᵥ1.7 in the dorsal root ganglia was evaluated using two distinct genome engineering constructs of the disclosure: KRAB-dCas9 and Zinc-Finger-KRAB proteins. It was found that the genome editing constructs of the disclosure were effective in suppressing acute and persistent nociceptive processing generated in animal models of peripheral inflammation and poly neuropathy. The genome editing constructs of the disclosure were discovered from testing multiple guide-RNAs (gRNAs) clones that were rationally designed using an *in silico* tool which predicts effective gRNAs based on chromatin position and sequence features into the split-dCas9 platform. Similarly, multiple ZFP-KRAB Naᵥ1.7 DNA targeting constructs were also discovered. The genome editing constructs of the disclosure were transfected into a murine neuroblastoma cell line that expresses Naᵥ1.7 (Neuro2a). Repression of Naᵥ1.7 was confirmed. Constructs showing the highest level of repression were chosen for subsequent *in vivo* studies.

Although other technologies, such as RNAi have been utilized to target Naᵥ1.7, studies have shown that the off-target effects of RNAi, as compared to CRISPRi, are far stronger and more pervasive than generally appreciated. In addition, as an exogenous system, CRISPR and ZFPs (unlike RNAi) do not compete with endogenous machinery such as microRNA or RISC complex function. Thus, RNAi can have an impact in the regular homeostatic mechanisms of RNA synthesis and degradation. In addition, CRISPR and ZFP methods target genomic DNA instead of RNA, which means that to achieve an effect, RNAi methods require a higher dosage with poorer pharmacokinetics prospects, as there is usually a high RNA turnover.

Studies have shown that partial repression of Naᵥ1.7 is sufficient to ameliorate pain. This knock down serves to produce a significant reversal of the hyperalgesia induced by hind paw inflammation. Using antisense oligonucleotides, mechanical pain could be ameliorated with 30 to 80% Naᵥ1.7 repression levels. Using microRNA 30b, around 50% repression of Naᵥ1.7 relieved neuropathic pain, while more recently microRNA182 ameliorated pain preventing Naᵥ1.7 overexpression in spared nerve injury rats. Similarly, shRNA mediated knockdown of Naᵥ1.7 prevented its overexpression in burn injury relieving pain. Other studies did not quantify the Naᵥ1.7 repression levels needed to reduce pain. Additionally, shRNA lentiviral vectors can reduce bone cancer pain by repressing Naᵥ1.7 40 to 60%.

The role of Naᵥ1.7 has been implicated in a variety of preclinical models, including those associated with robust inflammation as in the rodent carrageenan and CFA model. As such, the effect of knocking down Naᵥ1.7 in a paclitaxel-induced poly neuropathy was investigated using the genome editing constructs of the disclosure. Previous studies have shown that this treatment will induce Naᵥ1.7. Both epigenetic repressors ameliorate tactile allodynia to the same extent as the internal comparator gabapentin. Finally, the role of Naᵥ1.7 knock down using the genome editing constructs of the disclosure in hyperpathia induced by i.t. injection of BzATP was assessed. Spinal purine receptors have been shown to play an important role in the nociceptive processing initiated by a variety of stimulus conditions including inflammatory/incisional pain and a variety of neuropathies. The present studies indicate that repression of afferent Naᵥ1.7 expression in the nociceptor leads to a suppression of enhanced tactile sensitivity induced centrally. The mechanism underlying these results may reflect upon the observation that down regulation of Naᵥ1.7 in the afferent may serve to minimize the activation of microglia and astrocytes. These results suggest that, at least partially, pain signal transduction through Naᵥ1.7 is downstream of ATP signaling. Gabapentin was chosen as a positive control due to evidence that it decreases carrageenan-induced thermal hyperalgesia in rodents and because it is known to repress Naᵥ1.7. The results using the genome editing constructs of the disclosure are consistent with previous studies which have shown an inhibitory effect of gabapentin on Naᵥ1.7 expression levels, ultimately leading to a reduction of neuronal excitability.

The methods disclosed herein demonstrate the efficacy of spinal reduction in Naᵥ1.7 in three models of hyperpathia using the genome editing constructs disclosed herein. The studies presented herein, clearly establish significant target engagement and clear therapeutic efficacy with no evident adverse events after intrathecal knock down of Naᵥ1.7 when using the genome editing constructs of the disclosure. The role played by Naᵥ1.7 is in the nociceptive afferents, and their cell bodies are in the respective segmental DRG neurons. Accordingly, the DRG represents a target for this transfection motif. The intrathecal delivery route efficiently places AAVs to the DRG neurons which minimizes the possibility of off target biodistribution and reduces the viral load required to get transduction. In a particular embodiment, the genome editing constructs of the disclosure are administered intrathecally. Importantly, the relative absence of B and T cells in the cerebrospinal fluid, minimizes the potential immune response. In this regard, as ZFPs are engineered on human protein chasses, they intrinsically constitute a targeting approach with even lower potential immunogenicity. Indeed, a study in non-human primates (NHP) found that intrathecal delivery of a non-self protein (AAV9-GFP) produced immune responses which were not seen with the delivery of a self-protein.

The results presented herein, indicate that the genome editing constructs of the disclosure have favorable target engagement properties and efficacy for short- and longer-term time periods. As such, methods of using the genome editing constructs of the disclosure for sustained pain control are clearly indicated. As a potential clinical treatment, the genome editing constructs of the disclosure (*e.g.,* KRAB-dCas9 and ZFP-KRAB) show promise for treating chronic inflammatory and neuropathic pain. These systems allow for transient gene therapy, which is advantageous in the framework of chronic pain, as permanent pain insensitivity is not desired. While the treatment is transient, the weeks-long duration still presents a significant advantage compared to existing drugs which must be taken daily or hourly, and which may have undesirable addictive effects. Taken together, the results of these studies show a promising new avenue for treatment of chronic pain, a significant and increasingly urgent issue in our society. It should be noted that this therapeutic regimen addresses a critical pain phenotype: the enduring but reversible pain state. Chronic pain defined as pain states enduring greater than 3 months are not necessarily irreversible. Thanks to advances in medicine, the number of cancer survivors are steadily increasing in the last decades. This increase has led to a subsequent increase in the number of cancer-related side effects, and chemotherapy induced polyneuropathy is one of the most common adverse events. In this instance, a therapeutic approach that endures for months is preferable to one that is irreversible. Further, the use of multiple neuraxial interventions over time is a common motif for clinical interventions as with epidural steroids where repeat epidural delivery may occur over the year at several month intervals.

Accordingly, the disclosure provides an epigenetic approach to treat a subject with pain using constructs comprising a Zinc-Finger (ZF) fused to a repressor domain and/or dCas9 fused to a repressor domain, wherein dCas9 is a catalytically inactivated Cas9 that does not cleave DNA but maintains its ability to bind to the genome via a guide-RNA (gRNA). The epigenetic approaches described herein allow for transient gene therapy, which is advantageous in the framework of chronic pain, as permanent pain insensitivity is not desired. While the treatment is transient, the weeks-long duration still presents a significant advantage compared to existing drugs which must be taken daily or hourly, and which may have undesirable addictive qualities. Taken together, the epigenetic approaches using the ZF-repressor domain constructs and/or the dCas9-repressor domain constructs described herein provide new avenue for treatment of chronic pain, a significant and increasingly urgent issue in society.

In a further embodiment, the ZF-repressor domain construct and/or the dCas9-repressor domain construct is packaged and expressed by an adeno-associated virus (AAV). Use of such AAV systems are ideal for patients who would be administered the virus before a surgery, or for the use of chronic pain, in which the patient would have lowered pain for about a month at a time. In a particular embodiment, the AAV is AAV9. As the results indicate herein, the epigenetic approaches using the ZF-repressor domain constructs and/or the dCas9-repressor domain constructs of the disclosure were efficacious in an inflammatory chronic pain model and can be used for other pain modalities, including but not limited to, neuropathic pain, postoperative pain, migraine pain, and cancerinduced pain. In a further embodiment, the ZF-repressor domain constructs and/or dCas9-repressor domain constructs described herein can be designed to modulate additional genes, including but not limited to, Nav1.3, Nav1.9, TRPV1/2/3/4, Nav1.8, P2X4, P2X7, Atp1b3, Mapk8, Avpr1a, Calca, Htr1b, Oprm1, Mc1r, Kcnk9, KCNQ, TLR2/3.

The disclosure further provides for pharmaceutical compositions and formulations comprising a ZF-repressor domain construct and/or a dCas9-repressor domain construct described herein for specified modes of administration. In one embodiment a ZF-repressor domain construct and/or a dCas9-repressor domain construct described herein is an active ingredient in a composition comprising a pharmaceutically acceptable carrier. Such a composition is referred to herein as a pharmaceutical composition. A "pharmaceutically acceptable carrier" means any pharmaceutically acceptable means to mix and/or deliver the targeted delivery composition to a subject. The term "pharmaceutically acceptable carrier" as used herein means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be "acceptable" in the sense of being compatible with the other ingredients of the composition and is compatible with administration to a subject, for example a human. Such compositions can be specifically formulated for administration via one or more of a number of routes, such as the routes of administration described herein. Supplementary active ingredients also can be incorporated into the compositions. When an agent, formulation or pharmaceutical composition described herein, is administered to a subject, preferably, a therapeutically effective amount is administered. As used herein, the term "therapeutically effective amount" refers to an amount that results in an improvement or remediation of the condition.

Administration of the pharmaceutical composition to a subject is by means which the ZF-repressor domain construct and/or the dCas9-repressor domain construct contained therein will contact the target cell. The specific route will depend upon certain variables such as the target cell and can be determined by the skilled practitioner. Suitable methods of administering the ZF-repressor domain constructs and/or the dCas9-repressor domain constructs described herein to a patient include any route of *in vivo* administration that is suitable for delivering the ZF-repressor domain constructs and/or the dCas9-repressor domain constructs described herein to a patient. The preferred routes of administration will be apparent to those of skill in the art, depending on the preparation's type of viral gene therapy being used, the target cell population, and the disease or condition experienced by the subject. Typical methods of *in vivo* administration include, but are not limited to, intravenous administration, intraperitoneal administration, intrathecal administration, intramuscular administration, intracoronary administration, intracranial administration, intraarterial administration (*e.g.,* into a carotid artery), subcutaneous administration, transdermal delivery, intratracheal administration, subcutaneous administration, intraarticular administration, intraventricular administration, inhalation (*e.g.,* aerosol), intracerebral, nasal, oral, pulmonary administration, impregnation of a catheter, and direct injection into a tissue. In an embodiment where the target cells are in or near a tumor, a preferred route of administration is by direct injection into the tumor or tissue surrounding the tumor. For example, when the tumor is a breast tumor, the preferred methods of administration include impregnation of a catheter, and direct injection into the tumor.

Intravenous, intraperitoneal, intrathecal, intraganglionic, intraneural, intracranial and intramuscular administrations can be performed using methods standard in the art. Aerosol (inhalation) delivery can also be performed using methods standard in the art (see, for example, Stribling et al., Proc. Natl. Acad. Sci. USA 189: 11277-11281, 1992, which is incorporated herein by reference in its entirety). Oral delivery can be performed by complexing the zinc finger-repressor domain constructs described herein to a carrier capable of withstanding degradation by digestive enzymes in the gut of an animal. Examples of such carriers, include plastic capsules or tablets, such as those known in the art.

One method of local administration is by direct injection. Direct injection techniques are particularly useful for administering the ZF-repressor domain construct and/or the dCas9-repressor domain construct described herein to a cell or tissue that is accessible by surgery, and particularly, on or near the surface of the body. Administration of a composition locally within the area of a target cell refers to injecting the composition centimeters and preferably, millimeters from the target cell or tissue. For example, it was found herein that the intrathecal route of administration provided advantageous results.

The appropriate dosage and treatment regimen for the methods of treatment described herein will vary with respect to the particular disease being treated, the ZF-repressor domain constructs and/or the dCas9-repressor domain constructs described herein being delivered, and the specific condition of the subject. The skilled practitioner is to determine the amounts and frequency of administration on a case by case basis. In one embodiment, the administration is over a period of time until the desired effect (*e.g.,* reduction in symptoms is achieved). In a certain embodiment, administration is 1, 2, 3, 4, 5, 6, or 7 times per week. In a particular embodiment, administration is over a period of 1, 2, 3, 4, 5, 6, 7, 8, 9, or 10 weeks. In another embodiment, administration is over a period of 2, 3, 4, 5, 6 or more months. In yet another embodiment, treatment is resumed following a period of remission.

The ZF-repressor domain constructs and/or the dCas9-repressor domain constructs described herein can be administered in combination with one or more additional active agents that inhibit nociceptive pain signaling. For example, the ZF-repressor domain constructs and/or the dCas9-repressor domain constructs can be administered with one or more additional agents targeting single or multiple genes by delivering single or multiple gRNAs, or by designing ZFs that attach to single or multiple genes. Additional gene targets include, but are not limited to, P2x3, P2x4, P2x7, Nav1.3, capsaicin receptors (TRPV1/2/3/4), TRPA1, SHANK3, Voltage-gated Calcium channels (Cav2.2, Cav3.1, Cav3.2). FIG. 11 provides additional targets that can be inhibited in combination with the ZF-repressor domain constructs and/or the dCas9-repressor domain construct targets of the disclosure. Alternatively or in addition, various analgesics can be used in combination with the ZF-repressor domain constructs and/or the dCas9-repressor domain construct therapies of the disclosures. Such analgesics and other pain relief medicines are known in the art.

For use in the therapeutic applications described herein, kits and articles of manufacture are also described herein. Such kits can comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers can be formed from a variety of materials such as glass or plastic.

For example, the container(s) can comprise one or more ZF-repressor domain constructs and/or dCas9-repressor domain constructs described herein, optionally in a composition or in combination with another agent as disclosed herein. The container(s) optionally have a sterile access port (for example the container can be an intravenous solution bag or a vial having a stopper pierceable by a hypodermic injection needle). Such kits optionally comprise a compound disclosed herein with an identifying description or label or instructions relating to its use in the methods described herein.

A kit will typically comprise one or more additional containers, each with one or more of various materials (such as reagents, optionally in concentrated form, and/or devices) desirable from a commercial and user standpoint for use of a compound described herein. Non-limiting examples of such materials include, but are not limited to, buffers, diluents, filters, needles, syringes; carrier, package, container, vial and/or tube labels listing contents and/or instructions for use, and package inserts with instructions for use. A set of instructions will also typically be included.

A label can be on or associated with the container. A label can be on a container when letters, numbers or other characters forming the label are attached, molded or etched into the container itself; a label can be associated with a container when it is present within a receptacle or carrier that also holds the container, *e.g.,* as a package insert. A label can be used to indicate that the contents are to be used for a specific therapeutic application. The label can also indicate directions for use of the contents, such as in the methods described herein. These other therapeutic agents may be used, for example, in the amounts indicated in the Physicians' Desk Reference (PDR) or as otherwise determined by one of ordinary skill in the art.

In view of the foregoing and the following examples, the disclosure provides a number of aspect that are exemplified below:
Aspect 1. A recombinant gene repressor complex comprising a nuclease inactivated Cas9 (dCas9) protein fused to a transcription repressor and associated with at least one guide RNA (gRNA), wherein the gRNA specifically hybridizes to a target nucleic acid sequence encoding a gene product selected from the group consisting of TRPV1/2/3/4, P2XR3, TRPM8, TRPA1, P23X2, P2RY, BDKRB1/2, Hlr3A, ACCNs, TRPV4, TRPC/P, ACCN1/2, SCN1/3/8A/9A, SCN10A, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited.
Aspect 2. The recombinant gene repressor complex of aspect 1, wherein the target nucleic acid sequence is located on chromosome 2 at position 2q24.3.
Aspect 3. The recombinant gene repressor complex of aspect 1 or 2, wherein the gRNA comprises a sequence encoded by the sequence set forth in any one of 11-107.
Aspect 4. The recombinant gene repressor complex of any one of aspects 1 to 3, wherein the gRNA specifically hybridizes to a nucleic acid sequence encoding a SCN9A product (Nav1.7).
Aspect 5. The recombinant gene repressor complex of any one of aspects 1 to 4, wherein the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Aspect 6. The recombinant gene repressor complex of aspect 5, wherein the transcriptional repressor domain is a KRAB domain.
Aspect 7. A polynucleotide encoding one or more components of the recombinant gene repressor complex of any one of aspects 1 to 6.
Aspect 8. The polynucleotide of aspect 7, wherein the polynucleotide is codon optimized for expression in a human cell.
Aspect 9. A vector comprising a polynucleotide of aspect 7 or 8.
Aspect 10. The vector of aspect 9, wherein the polynucleotide is operably linked to a promoter.
Aspect 11. The vector of aspect 10, wherein the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/calmodulin-dependent protein kinase II promoter, a tubulin alpha I promoter, a neuron-specific enolase promoter and a glial fibrillary acidic protein (GFAP) promoter.
Aspect 12. The vector of aspect 9, wherein the vector comprises a polIII promoter upstream of the at least one guide RNA coding sequence.
Aspect 13. The vector of aspect 12, wherein the polIII promoter is selected from a U6 and H1 promoter.
Aspect 14. The vector of any one of aspect 9 to 13, further comprising a regulatory control sequence.
Aspect 15. The vector of aspect 14, wherein the regulatory control sequence is a woodchuck hepatitis virus posttranscriptional regulatory element (WPRE).
Aspect 16. The vector of any one of aspect 9-15, wherein the vector is a recombinant adeno-associated virus vector (rAAV vector).
Aspect 17. The vector of aspect 16, wherein the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
Aspect 18. The vector of aspect 16, wherein the polynucleotide includes one or more inverted repeats (ITRs).
Aspect 19. The vector of any one of aspects 9-18, wherein the polynucleotide or vector includes a poly A sequence.
Aspect 20. The vector of aspect 9, wherein the polynucleotide is engineered to express the one or more components in a cell.
Aspect 21. The vector of aspect 9, wherein the vector is a lentiviral vector, a gammaretroviral vector, or a herpes simplex viral vector.
Aspect 22. The vector of aspect 9, wherein the vector comprises a split dCas9 vector system.
Aspect 23. The vector of aspect 9 or 22, wherein the vector comprises a nucleic acid encoding a dCas9 having a sequence as set forth in SEQ ID NO:2.
Aspect 24. The vector of aspect 9 or 23, wherein the vector comprises a nucleic acid encoding a KRAB sequence of SEQ ID NO:7.
Aspect 25. The vector of any one of aspect 22 to 24, wherein the split vector system comprises a vector sequence selected from SEQ ID NO: 3, 4 and 10.
Aspect 26. A zinc-finger repressor construct comprising an engineered zinc finger DNA-binding domain coupled to a transcription repressor, wherein the zinc finger DNA-binding domain comprises one to six zinc-finger sequences and wherein the zinc finger sequences bind to a target nucleic acid sequence in a gene encoding a gene product selected from the group consisting of TRPV1/2/3/4, P2XR3, TRPM8, TRPA1, P23X2, P2RY, BDKRB1/2, Hlr3A, ACCNs, TRPV4, TRPC/P, ACCN1/2, SCN1/3/8A/9A, SCN10A, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited.
Aspect 27. The zinc-finger repressor construct of aspect 26, wherein the target nucleic acid sequence is a sequence set forth in Table 2.
Aspect 28. The zinc-finger repressor construct of aspect 26 or 27, wherein the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Aspect 29. A polynucleotide encoding the zinc-finger repressor construct of aspect 26, 27 or 28.
Aspect 30. The polynucleotide of aspect 29, wherein the polynucleotide is codon optimized for expression in a human cell.
Aspect 31. A vector containing the polynucleotide of aspect 29 or 30.
Aspect 32. The vector of aspect 31, wherein the polynucleotide is operably linked to a promoter.
Aspect 33. The vector of aspect 32, wherein the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/calmodulin-dependent protein kinase II promoter, a tubulin alpha I promoter, a neuron-specific enolase promoter and a glial fibrillary acidic protein (GFAP) promoter.
Aspect 34. The vector of aspect 31, 32 or 33, further comprising a regulatory control sequence.
Aspect 35. The vector of aspect 34, wherein the regulatory control sequence is a woodchuck hepatitis virus posttranscriptional regulatory element (WPRE).
Aspect 36. The vector of any one of aspects 31-35, wherein the vector is a recombinant adeno-associated virus vector (rAAV vector).
Aspect 37. The vector of aspect 36, wherein the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
Aspect 38. The vector of any one of aspects 31 to 37, wherein the polynucleotide includes one or more inverted repeats (ITRs) .
Aspect 39. The vector of any one of aspects 31-38, wherein the polynucleotide includes a poly A sequence.
Aspect 40. The vector of aspect 31, wherein the nucleic acid is engineered to express the one or more components in a cell.
Aspect 41. The vector of aspect 31, wherein the vector is a lentiviral vector, a gammaretroviral vector, or a herpes simplex viral vector.
Aspect 42. The vector of aspect 31, wherein the vector comprises a nucleic acid encoding a KRAB sequence of SEQ ID NO:7.
Aspect 43. An epigenetic-based method to treat or manage chronic pain in a subject comprising administering an effective amount of a complex of any one of aspect 1 to 6, a vector of any one of aspects 9 to 25, a construct of any one of aspects 26-28 or a vector of any one of aspects 31 to 42.
Aspect 44. An epigenetic-based method to treat or manage pain in a subject in need thereof, comprising administering an effective amount of a zinc finger-repressor construct and/or a dCas9-repressor domain complex to the subject, wherein dCas9 is catalytically inactivated Cas9 that does not cleave DNA but maintains its ability to bind to the genome via a guide-RNA (gRNA).
Aspect 45. The method of aspect 44, wherein the pain is selected from neuropathic pain, nociceptive pain, allodynia, inflammatory pain, inflammatory hyperalgesia, neuropathies, neuralgia, diabetic neuropathy, human immunodeficiency virus-related neuropathy, nerve injury, rheumatoid arthritic pain, osteoarthritic pain, burns, back pain, eye pain, visceral pain, cancer pain, bone cancer pain, migraine pain, pain from carpal tunnel syndrome, fibromyalgia pain, neuritis pain, sciatica pain, pelvic hypersensitivity pain, pelvic pain, post herpetic neuralgia pain, post-operative pain, post-stroke pain, and menstrual pain.
Aspect 46. The method of aspect 44, wherein in the pain is associated with a disease or disorder selected from the group consisting of neuropathic peripheral neuropathy, diabetic neuropathy, post herpetic neuralgia, trigeminal neuralgia, back injury, cancer neuropathy, HIV neuropathy, limb loss, carpal tunnel syndrome, stroke, alcoholism, hypothyroidism, uremia, multiple sclerosis, spinal cord injury, Parkinson's disease, and epilepsy.
Aspect 47. The epigenetic method of aspect 44, wherein the method is used to treat a subject with chronic pain.
Aspect 48. The epigenetic method of any one aspect 44 to 47, wherein the zinc finger-repressor construct comprises a repressor domain selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Aspect 49. The epigenetic method of aspect 48, wherein the repressor domain comprises KRAB.
Aspect 50. The epigenetic method of any one of aspect 44 to 49, wherein the zing finger-repressor construct binds to a target of Table 2.
Aspect 51. The epigenetic method of any one of aspect 44 to 47, wherein the dCas9-repressor domain complex comprises a repressor domain selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Aspect 52. The epigenetic method of aspect 51, wherein the repressor domain comprises KRAB.
Aspect 53. The epigenetic method of any one of aspect 44 to 47 or 51 to 52, wherein the dCas9-repressor domain construct comprises a guide RNA spacer sequence having a sequence selected from SEQ ID NOs:11-106 and 107.
Aspect 54. The epigenetic method of any one of aspects 44 to 53, wherein the zinc finger-repressor construct and/or the dCas9-repressor domain construct provides for non-permanent gene repression of a voltage gated sodium channel.
Aspect 55. The epigenetic method of aspect 54, wherein the voltage gated sodium channel is selected from NaV1.7, NaV1.8, and NaV1.9.
Aspect 56. The epigenetic method of aspect 55, wherein the voltage gated sodium channel is NaV1.7.
Aspect 57. The epigenetic method of any one of aspects 44-56, wherein the zinc finger-repressor construct and/or the dCas9-repressor domain construct is packaged and delivered by a recombinant virus or vector.
Aspect 58. The epigenetic method of aspect 57, wherein the recombinant virus is an adenovirus, gammaretrovirus, adeno-associated virus (AAV), herpes simplex virus (HSV) or lentivirus.
Aspect 59. The epigenetic method of aspect 57, wherein the recombinant virus is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
Aspect 60. The epigenetic method of any one of aspect 44 to 59, wherein the zinc finger-repressor construct and/or the dCas9-repressor domain construct is administered intravenous, intraperitoneal, intrathecal, intraganglionic, intraneural, intracranial or intramuscular.

The following examples are intended to illustrate but not limit the disclosure. While they are typical of those that might be used, other procedures known to those skilled in the art may alternatively be used.

### EXAMPLES

### Example 1

**Vector Design and Construction.** Cas9 and Zinc-Finger AAV vectors were constructed by sequential assembly of corresponding gene blocks (Integrated DNA Technologies) into a custom synthesized rAAV2 vector backbone. gRNA sequences were inserted into dNCas9 plasmids by cloning oligonucleotides (IDT) encoding spacers into AgeI cloning sites via Gibson assembly. gRNAs were designed utilizing an *in silico* tool to predict gRNAs73.

**Mammalian Cell Culture.** Neuro2a cells were grown in EMEM supplemented with 10% fetal bovine serum (FBS) and 1% Antibiotic-Antimycotic (Thermo Fisher Scientific) in an incubator at 37°C and 5% CO2 atmosphere.

**Lipid-Mediated Cell Transfections.** One day prior to transfection, Neuro2a cells were seeded in a 24-well plate at a cell density of 1 or 2E+5 cells per well. 0.5 µg of each plasmid was added to 25 µL of Opti-MEM medium, followed by addition of 25 µL of Opti-MEM containing 2 µL of Lipofectamine 2000. The mixture was incubated at room temperature for 15 min. The entire solution was then added to the cells in a 24-well plate and mixed by gently swirling the plate. Media was changed after 24 h, and the plate was incubated at 37°C for 72 h in a 5% CO2 incubator. Cells were harvested, spun down, and frozen at 80°C.

**Production of AAVs.** Virus was prepared by the Gene Transfer, Targeting and Therapeutics (GT3) core at the Salk Institute of Biological Studies (La Jolla, CA) or in-house utilizing the GT3 core protocol. Briefly, AAV2/1, AAV2/5, and AAV2/9 virus particles were produced using HEK293T cells via the triple transfection method and purified via an iodixanol gradient. Confluency at transfection was between 80% and 90%. Media was replaced with pre-warmed media 2h before transfection. Each virus was produced in five 15 cm plates, where each plate was transfected with 10 µg of pXR-capsid (pXR-1, pXR-5, and pXR-9), 10 of µg recombinant transfer vector, and 10 µg of pHelper vector using polyethylenimine (PEI; 1 mg/mL linear PEI in DPBS [pH 4.5], using HCl) at a PEI:DNA mass ratio of 4:1. The mixture was incubated for 10 min at room temperature and then applied dropwise onto the media. The virus was harvested after 72 h and purified using an iodixanol density gradient ultracentrifugation method. The virus was then dialyzed with 1x PBS (pH 7.2) supplemented with 50 mM NaCl and 0.0001% of Pluronic F68 (Thermo Fisher Scientific) using 50-kDa filters (Millipore) to a final volume of ~100 µL and quantified by qPCR using primers specific to the ITR region, against a standard (ATCC VR-1616): AAV-ITR-F: 5' -CGGCCTCAGTGAGCGA-3' (SEQ ID NO:127) and AAV-ITR-R: 5' -GGAACCCCTAGTGATGGAGTT-3'(SEQ ID NO:128).

**Animals Experiments.** All animal procedures were performed in accordance with protocols approved by the Institutional Animal Care and Use Committee (IACUC) of the University of California, San Diego. All mice were acquired from Jackson Laboratory. Two-month-old adult male C57BL/6 mice (25-30g) were housed with food and water provided ad libitum, under a 12 h light/dark cycle with up to 5 mice per cage. All behavioral tests were performed during the light cycle period.

**Intrathecal AAV Injections.** Anesthesia was induced with 2.5% isoflurane delivered in equal parts O2 and room air in a closed chamber until a loss of the righting reflex was observed. The lower back of mice was shaven and swabbed with 70% ethanol. Mice were then intrathecally (i.t.) injected using a Hamilton syringe and 30G needle as previously described104 between vertebrae L4 and L5 with 5 µL of AAV for a total of 1E+12 vg/mouse. A tail flick was considered indicative of appropriate needle placement. Following injection, all mice resumed motor activity consistent with that observed prior to i.t. injection.

**Pain Models.** Intraplantar carrageenan injection: Carrageenan-induced inflammation is a classic model of edema formation and hyperalgesia105-107. 21 days after AAV pre-treatment, anesthesia was induced as described above. Lambda carrageenan (Sigma Aldrich; 2% (W/V) dissolved in 0.9% (W/V) NaCl solution, 20 µL) was subcutaneously injected with a 30G needle into the plantar (ventral) surface of the ipsilateral paw. An equal amount of isotonic saline was injected into the contralateral paw. Paw thickness was measured with a caliper before and 4h after carrageenan/saline injections as an index of edema/inflammation. Hargreaves testing was performed before injection (t=0) and (t= 30, 60, 120, 240 minutes and 24 hours post-injection). The experimenter was blinded to the composition of treatment groups. Mice were euthanized after the 24-hour time point.

Paclitaxel-induced neuropathy: Paclitaxel (Tocris Biosciences, 1097) was dissolved in a mixture of 1:1:18 [1 volume ethanol/1 volume Cremophor EL (Millipore, 238470)/18 volumes of sterilized 0.9% (W/V) NaCl solution]. Paclitaxel injections (8 mg/kg) were administered intraperitoneally (i.p.) in a volume of 1 mL/100 g body weight every other day for a total of four injections to induce neuropathy (32 mg/kg), resulting in a cumulative human equivalent dose of 28.4-113.5 mg/m2 as previously described67. Behavioral tests were performed 24 hours after the last dosage.

Intrathecal BzATP injection: BzATP (2'(3')-O-(4-Benzoylbenzoyl) adenosine 5'-triphosphate triethylammonium salt) was purchased from Millipore Sigma and, based on previous tests, was dissolved in saline (NaCl 0.9%) to final a concentration of 30 nmol. Saline solution was also used as a vehicle control and both were delivered in a 5 µL volume. Intrathecal injections were performed under isoflurane anesthesia (2.5%) by lumbar puncture with a 30-gauge needle attached to a Hamilton syringe.

**Behavioral tests.** Mice were habituated to the behavior and to the experimental chambers for at least 30 min before testing. As a positive control, gabapentin (Sigma, G154) was dissolved in saline solution and injected i.p. at 100 mg/kg/mouse.

Thermal Withdrawal Latency (Hargreaves Test): To determine the acute nociceptive thermal threshold, the Hargreaves' test was conducted using a plantar test device (Ugo Basile, Italy)108. Animals were allowed to freely move within a transparent plastic enclosement (6 cm diameter × 16 cm height) on a glass floor 40 min before the test. A mobile radiant heat source was then placed under the glass floor and focused onto the hind paw. Paw withdrawal latencies were measured with a cutoff time of 30 seconds. An IR intensity of 40 was employed. The heat stimulation was repeated three times on each hind paw with a 10 min interval to obtain the mean latency of paw withdrawal. The experimenter was blinded to composition of treatment groups.

Tactile allodynia: For the BzATP pain model, tactile thresholds (allodynia) were assessed 30 minutes, 1, 2, 3, 6, 24 hours after the BzATP injection. For the Paclitaxel model, tactile thresholds (allodynia) were assessed 24 hours and 29 days after the last Paclitaxel injection. Forty-five minutes before testing, mice were placed in clear plastic wire mesh-bottom cages for acclimation. The 50% probability of withdrawal threshold was assessed using von Frey filaments (Seemes Weinstein von Frey anesthesiometer; Stoelting Co., Wood Dale, IL, USA) ranging from 2.44 to 4.31 (0.04-2.00 g) in an up-down method, as previously described107.

**Cold allodynia:** Cold allodynia was measured by applying drops of acetone to the plantar surface of the hind paw. Mice were placed in individual plastic cages on an elevated platform and were habituated for at least 30 min until exploratory behaviors ceased. Acetone was loaded into a one mL syringe barrel with no needle tip. One drop of acetone (approximately 20 µL) was then applied through the mesh platform onto the plantar surface of the hind paw. Care was taken to gently apply the bubble of acetone to the skin on the paw without inducing mechanical stimulation through contact of the syringe barrel with the paw. Paw withdrawal time in a 60s observation period after acetone application was recorded. Paw withdrawal behavior was associated with secondary animal responses, such as rapid flicking of the paw, chattering, biting, and/or licking of the paw. Testing order was alternated between paws (i.e. right and left) until five measurements were taken for each paw. An interstimulation interval of 5 minutes was allowed between testing of right and left paws.

**Tissue collection.** After the 24-hour time carrageenan time point, spinal cords were collected via hydroextrusion (injection of 2 mL of iced saline though a short blunt 20 gauge needle placed into the spinal canal following decapitation). After spinal cord tissue harvest, the L4-L6 DRG on each side were combined and frozen as for the spinal cord. Samples were placed in Dnase/Rnase-free 1.5 mL centrifuge tubes, quickly frozen on dry ice, and then stored at 80°C for future analysis.

**Gene Expression Analysis and qPCR.** RNA from Neuro2a cells was extracted using Rneasy Kit (QIAGEN; 74104) and from DRG using Rneasy Micro Kit (QIAGEN; 74004). cDNA was synthesized from RNA using Protoscript II Reverse Transcriptase Kit (NEB; E6560L). Real-time PCR (qPCR) reactions were performed using the KAPA SYBR Fast qPCR Kit (Kapa Biosystems; KK4601), with gene-specific primers in technical triplicates and in biological triplicates (Neuro2a cells). Relative mRNA expression was normalized to GAPDH levels and fold change was calculated using the comparative CT (ΔΔCT) method and normalized to GAPDH. Mean fold change and SD were calculated using Microsoft Excel.

**Western Blot.** Neuro2a cells were thawed and protein extraction was performed with RIPA buffer (25mM Tris•HCl pH 7.6, 150mM NaCl, 1% NP-40, 1% sodium deoxycholate, 0.1% SDS; Thermo Fisher 89900) supplemented with protease inhibitors (Sigma P8849). Total protein was quantified with BCA protein assay kit (Thermo Fisher 23225), and 40 µg of protein were loaded into 4-15% polyacrylamide gels (BioRad 4561085). Proteins were transfer to a PVDF membrane (Thermo Fisher IB401001) and the membrane was blocked with 5% (W/V) blotting-grade blocker (Biorad 1706404) dissolved in TBS-T (Thermo Fisher, 28358 supplemented with 0.1% (V/V) Tween-20; BioRad 1610781). Membranes were then incubated overnight at 4°C with primary antibodies: anti-NaV1.7 diluted 1:1000 (Abcam; ab85015) and anti-GAPDH (Cell Signaling, 2118) diluted 1:4000. Membranes were then washed three times with TBS-T and incubated for 1 h at room temperature with anti-rabbit horseradish-peroxidase-conjugated secondary antibody (Cell Signaling, 7074) diluted 1:20000. After being washed with TBST, blots were visualized with SuperSignal West Femto Chemiluminescent Substrate (Thermo Fisher) and visualized on an X-ray film.

**RNAscope ISH Assays.** The mCherry, NaV1.7, and NeuN probes were designed by Advanced Cell Diagnostics (Hayward, CA). The mCherry probe (ACD Cat# 404491) was designed to detect 1480-2138 bp (KF450807.1, C1 channel), the NaV1.7 (ACD Cat#313341), was designed to detect 3404-4576 bp of the Mus musculus NaV1.7 mRNA sequence (NM_018852.2, C3 channel), and the NeuN probe (ACD Cat# 313311) was designed to target the 1827-3068 bp of the Mus musculus Neun gene (NM_001039167.1, C2 channel). Before sectioning, DRG were placed into 4% PFA for 2 hours at room temperature, followed by incubation in 30% sucrose overnight at 4°C. Tissues were sectioned (12 µm thick) and mounted on positively charged microscopic glass slides (Fisher Scientific). All hybridization and amplification steps were performed following the ACD RNAscope V2 fixed tissue protocol. Stained slides were coverslipped with fluorescent mounting medium (ProLong Gold Anti-fade Reagent P36930; Life Technologies) and scanned into digital images with a Zeiss 880 Airyscan Confocal at 20x magnification. Data was processed using ZEN software (manufacturer-provided software).

**Statistical analysis.** Results are expressed as mean +/standard error (SE). Statistical analysis was performed using GraphPad Prism (version 8.0, GraphPad Software, San Diego, CA, USA). Results were analyzed using Student's t-test (for differences between two groups), one-way ANOVA (for multiple groups), or two-way ANOVA with the Bonferroni post hoc test (for multiple groups time-course experiments). Differences between groups with p < 0.05 were considered statistically significant.

**Split Vector Design and Construction.** Split-Cas9/dCas9 AAV vectors were constructed by sequential assembly of corresponding gene blocks (Integrated DNA Technologies) into a custom synthesized rAAV2 vector backbone. gRNA sequences were inserted into NCas9 or dNCas9 plasmids by cloning oligonucleotides (IDT) encoding spacers into AgeI cloning sites via Gibson assembly.

**AAV Production.** Briefly, AAV2/9, virus particles were produced using HEK293T cells via the triple transfection method and purified via an iodixanol gradient. Confluency at transfection was between 80% and 90%. Media was replaced with pre-warmed media 2 hr before transfection. Each virus was produced in 5x15 cm plates, where each plate was transfected with 7.5 mg of pXR-capsid (pXR-9), 7.5 of mg recombinant transfer vector, and 22.5 mg of pAd5 helper vector using polyethylenimine (PEI; 1 mg/mL linear PEI in 1DPBS [pH 4.5], using HCl) at a PEI:DNA mass ratio of 4:1. The mixture was incubated for 10 min at room temperature and then applied dropwise onto the media. The virus was harvested after 72 hr and purified using an iodixanol density gradient ultracentrifugation method. The virus was then dialyzed with 1 PBS (pH 7.2) supplemented with 50 mM NaCl and 0.0001% of Pluronic F68 (Thermo Fisher Scientific) using 100-kDa filters (Millipore) to a final volume of 1 mL.

**AAV injections.** AAV injections were done in adult C57BL/6J mice (10 weeks) via intrathecal injections using 1E+12 vg/mouse of each split-Cas9 (total virus of 2E+12 vg/mouse) or 1E+12 for ZF injections.

To establish robust Naᵥ1.7 repression, *in vitro* repression efficacy of Naᵥ1.7 using KRAB-dCas9 and ZFP-KRAB constructs were compared. Towards this, ten guide-RNAs (gRNAs; **Table 3)** - designed by an *in silico* tool that predicts highly effective gRNAs based on chromatin position and sequence features - were cloned into a split-dCas9 platform. In addition, two gRNAs that were predicted to have the highest efficiency (SCN9A-1 and SCN9A-2) were also cloned into a single construct, since higher efficacy can be achieved by using multiple gRNAs. Next, four ZFP-KRAB constructs targeting the Naᵥ1.7 DNA sequence were designed **(Table 4).** These constructs were transfected into a mouse neuroblastoma cell line that expresses Naᵥ1.7 (Neuro2a) and Naᵥ1.7 was repressed relative to GAPDH with qPCR. Six of ten gRNAs repressed the Naᵥ1.7 transcript by >50% compared to the non-targeting gRNA control, with gRNA-2 being the single gRNA having the highest repression (56%) and with the dual-gRNA having repression levels of 710 (p < 0.0001); these were utilized for subsequent *in vivo* studies **(****Figure 5A****).** Of the ZFP-KRAB designs, the Zinc-Finger-4-KRAB construct had the highest repression (88%; p < 0.0001) compared to the negative control (mCherry), which was selected for subsequent *in vivo* studies **(****Figure 5A****).** Western blotting confirmed a corresponding decrease in protein level for both the Zinc-Finger-4-KRAB and KRAB-dCas9-dual-gRNA groups **(****Figure 5B****)** .

**Table 3: CRISPR-Cas9 guide RNA spacer sequences**

| **gRNA** | **Sequence** |
|---|---|
| SCN9A-1 | ACAGTGGGCAGGATTGAAA (SEQ ID NO:129) |
| SCN9A-2 | GCAGGTGCACTCACCGGGT (SEQ ID NO:130) |
| SCN9A-3 | GAGCTCAGGGAGCATCGAGG (SEQ ID NO:131) |
| SCN9A-4 | AGAGTCGCAATTGGAGCGC (SEQ ID NO:132) |
| SCN9A-5 | CCAGACCAGCCTGCACAGT (SEQ ID NO:133) |
| SCN9A-6 | GAGCGCAGGCTAGGCCTGCA (SEQ ID NO:134) |
| SCN9A-7 | CTAGGAGTCCGGGATACCC (SEQ ID NO:135) |
| SCN9A-8 | GAATCCGCAGGTGCACTCAC (SEQ ID NO:136) |
| SCN9A-9 | GACCAGCCTGCACAGTGGGC (SEQ ID NO:137) |
| SCN9A-10 | GCGACGCGGTTGGCAGCCGA (SEQ ID NO:138) |

**Table 4: Zinc finger protein genomic target sequences**

| **ZF Name** | **ZF Target Sequence** |
|---|---|
| ZF1 | GGCGAGGTGATGGAAGGG (SEQ ID NO:139) |
| ZF2 | GAGGGAGCTAGGGGTGGG (SEQ ID NO:140) |
| ZF3 | AGTGCTAATGTTTCCGAG (SEQ ID NO:141) |
| ZF4 | TAGACGGTGCAGGGCGGA (SEQ ID NO:142) |

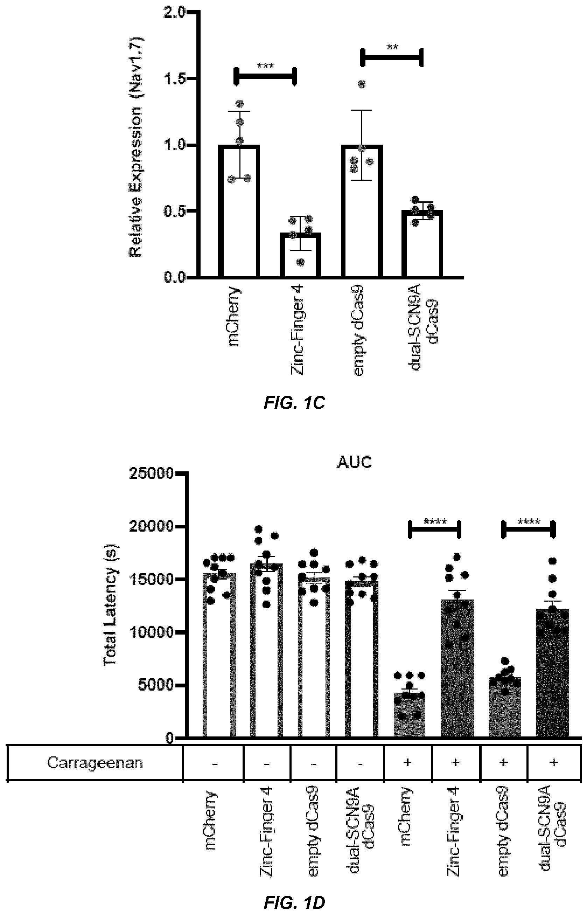

Having established *in vitro* Naᵥ1.7 repression, testing the effectiveness of the best ZFP-KRAB and KRAB-dCas9 constructs from the *in vitro* screens (Zinc-Finger-4-KRAB and KRAB-dCas9-dual-gRNA) in a carrageenan-induced model of inflammatory pain was performed. Mice were intrathecally (i.t.) injected with 1E+12 vg/mouse of AAV9-mCherry (negative control; n=10), AAV9-Zinc-Finger-4-KRAB (n=10), AAV9-KRAB-dCas9-no-gRNA (negative control; n=10) and AAV9-KRAB-dCas9-dual-gRNA (n=10). The intrathecal delivery of AAV9, which has significant neuronal tropism, serves to efficiently target DRG **(****Figure 6A****).** After 21 days, thermal pain sensitivity was measured to establish a baseline response threshold. Inflammation was induced in all four groups of mice by injecting one hind paw with carrageenan (ipsilateral), while the other hind paw (contralateral) was injected with saline to serve as an in-mouse control. Mice were then tested for thermal pain sensitivity at 30 minutes, 1, 2, 4, and 24 hours after carrageenan injection **(****Figure 1B****).** Twenty-four hours after carrageenan administration, mice were euthanized and DRG (L4-L6) were extracted. The expression levels of Naᵥ1.7 was determined by qPCR, and a significant repression of Naᵥ1.7 was observed in mice injected with AAV9-Zinc-Finger-4-KRAB (67%; p=0.0008) compared to mice injected with AAV9-mCherry, and in mice injected with AAV9-KRAB-dCas9-dual-gRNA (50%; p=0.0033) compared to mice injected with AAV9-KRAB-dCas9-no-gRNA **(****Figure 1C****).** The mean paw withdrawal latencies (PWL) were calculated for both carrageenan and saline injected paws **(**Figure 6B-C) and the area under the curve (AUC) for the total mean PWL was calculated. As expected, compared to saline-injected paws, carrageenan-injected paws developed thermal hyperalgesia, measured by a decrease in PWL after application of a thermal stimulus **(****Figure 1D****).** In addition, a significant increase in PWL in mice injected with either AAV9-Zinc-Finger-4-KRAB or AAV9-KRAB-dCas9-dual-gRNA was observed, indicating that the repression of Naᵥ1.7 in mouse DRG leads to lower thermal hyperalgesia in an inflammatory pain state. The thermal latency of the control (un-inflamed paw) was not significantly different across AAV treatment groups, indicating that the knock down of the Naᵥ1.7 had minimal effect upon normal thermal sensitivity. As an index of edema/inflammation, the ipsilateral and contralateral paws were measured with a caliper before and 4 hours after carrageenan injection, which is the time point with the highest thermal hyperalgesia. Significant edema formation was observed in both experimental and control groups, indicating that Naᵥ1.7 repression has no effect on inflammation **(****Figure 6D****).**

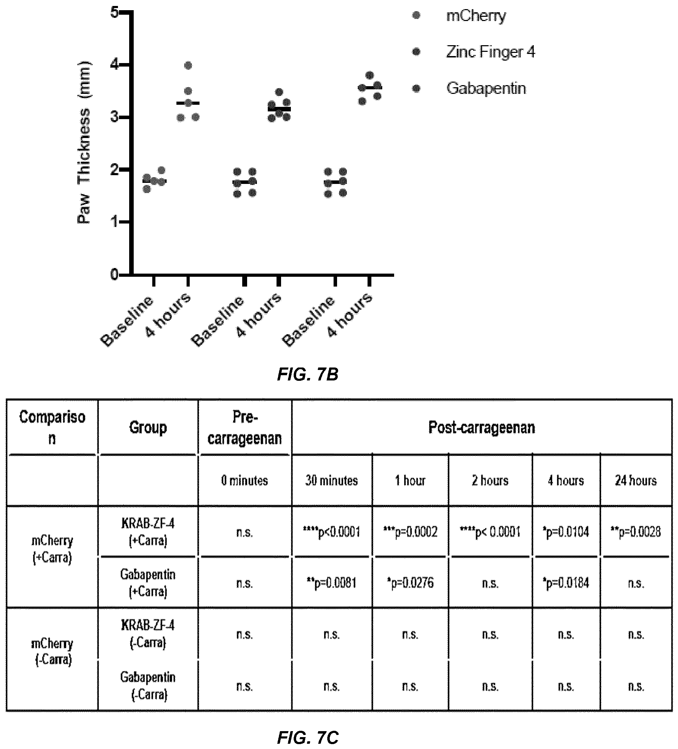

To validate the efficacy of ZFP-KRAB in ameliorating thermal hyperalgesia in a carrageenan model of inflammatory pain, a separate experiment was conducted to test the small molecule drug gabapentin as a positive control. Mice were i.t. injected with 1E+12 vg/mouse of AAV9-mCherry (n=5), AAV9-Zinc-Finger-4-KRAB (n=6), or saline (n=5). After 21 days, thermal nociception was measured in all mice as previously described. One hour before carrageenan administration, the mice that received intrathecal saline were injected as a positive comparator with intraperitoneal (i.p.) gabapentin (100 mg/kg). This agent is known to reduce carrageenan-induced thermal hyperalgesia in rodents through binding to spinal alpha2 delta subunit of the voltage gated calcium channel. Twenty-four hours after carrageenan administration, mice were euthanized and DRG (L4-L6) were extracted. The expression levels of Naᵥ1.7 were determined by qPCR, and a significant repression of Naᵥ1.7 was observed in AAV9-Zinc-Finger-4-KRAB (***p=0.0007) and in the gabapentin groups (*p=0.0121) **(****Figure 7A****).** The ipsilateral and contralateral paws were measured with a caliper before and 4 hours after carrageenan injection, and confirmed significant edema formation in the injected paw of all groups as compared to the non-injected paw in all groups **(****Figure 7B****).** The mean PWL was calculated for both carrageenan and saline injected paws **(**Figure 2B-C). Paw withdrawal latencies of carrageenan injected paws for AAV9-Zinc-Finger-4-KRAB and gabapentin groups were then compared at each time point to the AAV9-mCherry carrageenan injected control using a two-way ANOVA calculation to determine whether there was any significant reduction in thermal hyperalgesia (Figure **7C****).** When comparing carrageenan-injected hind paws, it was observed that only AAV9-Zinc-Finger-4-KRAB had significantly higher PWL at all the time points following carrageenan injection when compared to the AAV9-mCherry control. In addition, significance was observed in PWL for the gabapentin positive control group at the 30 minute, 1 hour, and four hour time points, but not the 24 hour time point. This result reflects the half-life of gabapentin (3-5 hours). The area under the curve (AUC) was then calculated for thermal hyperalgesia. A significant increase was observed in PWL in the carrageenan-injected gabapentin group (p=0.0208) **(****Figure 2B****),** and in the Zinc-Finger-4-KRAB group (115% improvement, p=0.0021) **(****Figure 2C****)** compared to the carrageenan-injected AAV9-mCherry control. In addition, the AAV9-Zinc-Finger-4-KRAB group had 31% higher PWL than the gabapentin positive control group. Of note, the thermal escape latency of the contralateral non-inflamed paw showed no significant difference among groups.

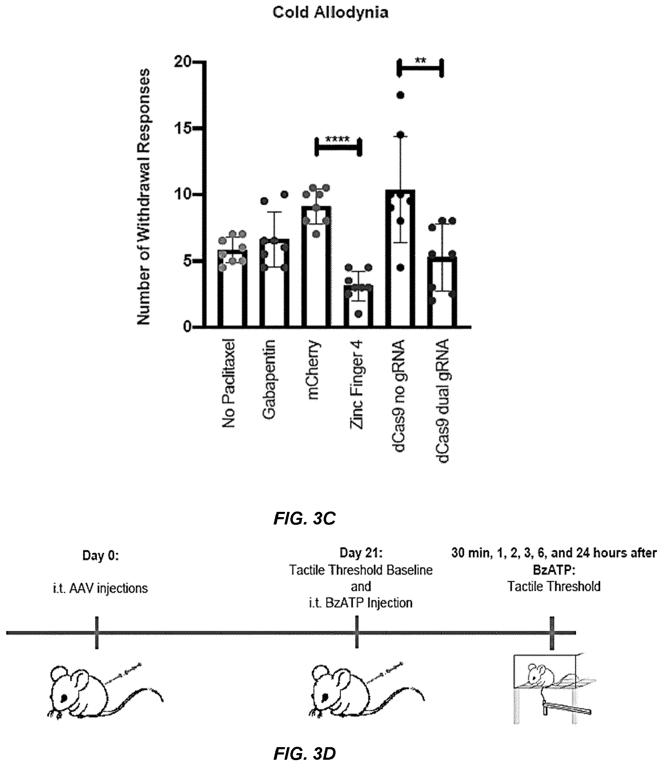

After having established *in vivo* efficacy in an inflammatory pain model, experiments were performed to validate the epigenome repression strategy for neuropathic pain using the polyneuropathy model by the chemotherapeutic paclitaxel. To establish this model, mice were first injected with 1E+12 vg/mouse of AAV9-mCherry (n=8), AAV9-Zinc-Finger-4-KRAB (n=8), AAV9-KRAB-dCas9-dual-gRNA (n=8), AAV9-KRAB-dCas9-no-gRNA (n=8), or saline (n=16). 14 days later and before paclitaxel administration, a baseline for tactile threshold (von Frey filaments) was established. Mice were then administered paclitaxel at days 14, 16, 18, and 20, with a dosage of 8 mg/kg (total cumulative dosage of 32 mg/kg), with a group of saline injected mice not receiving any paclitaxel (n=8) to establish the tactile allodynia caused by the chemotherapeutic. 21 days after the initial injections and one hour before testing, a group of saline injected mice (n=8) were injected with i.p. gabapentin (100 mg/kg). Mice were then tested for tactile allodynia via von Frey filaments and for cold allodynia via acetone testing **(****Figure 3A****).** A 50% tactile threshold was calculated. A decrease in tactile threshold was observed in mice receiving AAV9-mCherry and AAV9-KRAB-dCas9-no-gRNA, while mice that received gabapentin, AAV9-Zinc-Finger-4-KRAB, and AAV9-KRAB-dCas9-dual-gRNA had increased withdrawal thresholds, indicating that *in situ* Naᵥ1.7 repression leads to amelioration in chemotherapy induced tactile allodynia **(****Figure 3B****).** Similarly, an increase in the number of withdrawal responses is seen in mice tested for cold allodynia in the negative control groups (AAV9-mCherry and AAV9-KRAB-dCas9-no-gRNA), while both AAV9-Zinc-Finger-4-KRAB and AAV9-KRAB-dCas9-dual-gRNA groups had a decrease in withdrawal responses, indicating that *in situ* repression of Naᵥ1.7 also leads to a decrease in chemotherapy induced cold allodynia **(****Figure 3C****).**

Experiments were then performed to test whether *in situ* repression of Naᵥ1.7 via KRAB-dCas9 could ameliorate neuropathic pain induced by BzATP. This molecule activates P2X receptors located on central terminals leading to a centrally mediated hyperalgesic state. Mice were first injected with 1E+12 vg/mouse of AAV9-mCherry (n=6), AAV9-KRAB-dCas9-no-gRNA (n=5), and AAV9-KRAB-dCas9-dual-gRNA (n=6). After 21 days, tactile thresholds were determined with von Frey filaments, and mice were injected i.t. with BzATP (30 nmol). Tactile allodynia was then measured at 30 min, 1, 2, 3, 6, and 24 hours after BzATP administration **(****Figure 3D****).** A significant decrease in tactile allodynia was observed at 30 min, 1 and 2 hour time points in mice injected with AAV9-KRAB-dCas9-dual-gRNA, and an overall increase in tactile threshold at all time points **(****Figure 3E****)** .

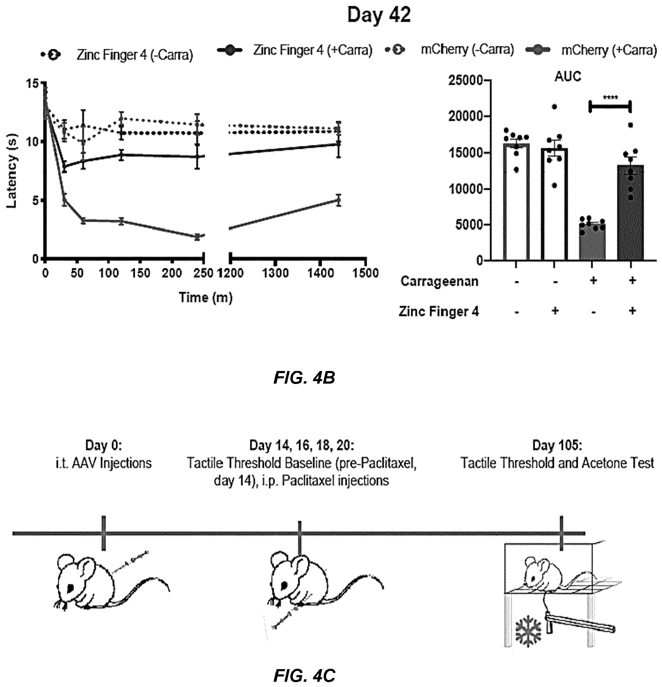

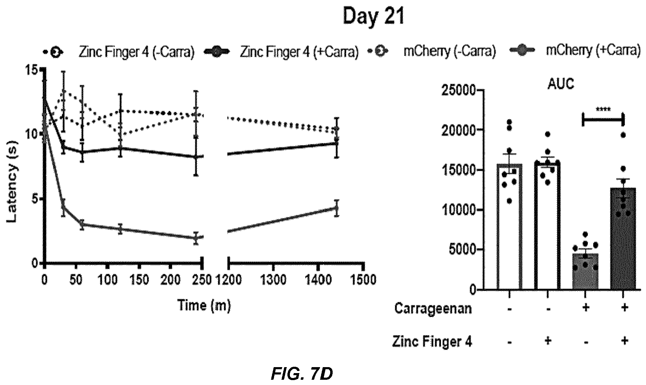

To determine whether *in situ* repression of Naᵥ1.7 was efficacious long-term, the carrageenan inflammatory pain model was repeated with thermal hyperalgesia at 21 and 42 days after i.t. AAV injection (n=8/group) **(****Figure 4A****).** A significant improvement in PWL was observed for carrageenan-injected paws in Zinc-Finger-4-KRAB groups at both day 21 **(****Figure 7D****)** and day 42 **(****Figure 4B****)** demonstrating the durability of this approach. To determine whether *in situ* repression of Naᵥ1.7 was also efficacious long-term in a poly-neuropathic pain model, tactile and cold allodynia was measured 49 days after initial AAV injections and 29 days after the last paclitaxel injection (total cumulative dosage of 32 mg/kg; **Figure 4C****).** Compared to the earlier time point **(**Figure 3B-C), mice from both AAV9-mCherry (n=8) and AAV9-KRAB-dCas9-dual-gRNA (n=8) groups had increased tactile allodynia at day 49 as compared to day 21, and responded to the lowest von Frey filament examined (0.04 g). In comparison, mice receiving AAV9-Zinc-Finger-4-KRAB and AAV9-KRAB-dCas9-dual-gRNA had increased withdrawal thresholds, indicating that *in situ* Naᵥ1.7 repression leads to long-term amelioration in chemotherapy-induced tactile allodynia **(****Figure 4C****).** As before, an increase in the number of withdrawal responses is seen in mice tested for cold allodynia in the negative control groups (AAV9-mCherry and AAV9-KRAB-dCas9-no-gRNA), while both AAV9-Zinc-Finger-4-KRAB and AAV9-KRAB-dCas9-dual-gRNA groups had a decrease in withdrawal responses, indicating that *in situ* repression of Naᵥ1.7 also leads long-term amelioration of chemotherapy induced cold allodynia **(****Figure 4E****).**

The disclosure also demonstrates that the methods and compositions can reverse the chronic pain state. In these experiments mice were first treated with paclitaxel to induce chronic pain. After confirming mechanical allodynia with von Frey filaments, mice were then injected with the gene repression therapy and two and three weeks after there was a reversal in mechanical allodynia with the group of mice that received ZF gene repression therapy **(**FIG. 10A-B).

It will be understood that various modifications may be made without departing from the spirit and scope of this disclosure. Accordingly, other embodiments are within the scope of the following claims.

### Embodiments

Embodiment 1. A recombinant gene repressor complex comprising
   a nuclease inactivated Cas9 (dCas9) protein fused to a transcription repressor and associated with at least one guide RNA (gRNA), wherein the gRNA specifically hybridizes to a target nucleic acid sequence encoding a gene product selected from the group consisting of TRPV1/2/3/4, P2XR3, TRPM8, TRPA1, P23X2, P2RY, BDKRB1/2, Hlr3A, ACCNs, TRPV4, TRPC/P, ACCN1/2, SCN1/3/8A/9A, SCN10A, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited.
Embodiment 2. The recombinant gene repressor complex of Embodiment 1, wherein the target nucleic acid sequence is located on chromosome 2 at position 2q24.3.
Embodiment 3. The recombinant gene repressor complex of Embodiment 1 or 2, wherein the gRNA comprises a sequence encoded by the sequence set forth in any one of 11-107.
Embodiment 4. The recombinant gene repressor complex of any one of Embodiments 1 or 2, wherein the gRNA specifically hybridizes to a nucleic acid sequence encoding a SCN9A product (Nav1.7).
Embodiment 5. The recombinant gene repressor complex of any one of Embodiments 1 or 2, wherein the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Embodiment 6. The recombinant gene repressor complex of Embodiment 5, wherein the transcriptional repressor domain is a KRAB domain.
Embodiment 7. A polynucleotide encoding one or more components of the recombinant gene repressor complex of Embodiment 1.
Embodiment 8. The polynucleotide of Embodiment 7, wherein the polynucleotide is codon optimized for expression in a human cell.
Embodiment 9. A vector comprising a polynucleotide of Embodiment 7 or 8.
Embodiment 10. The vector of Embodiment 9, wherein the polynucleotide is operably linked to a promoter.
Embodiment 11. The vector of Embodiment 10, wherein the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/calmodulin-dependent protein kinase II promoter, a tubulin alpha I promoter, a neuron-specific enolase promoter and a glial fibrillary acidic protein (GFAP) promoter.
Embodiment 12. The vector of Embodiment 9, wherein the vector comprises a polIII promoter upstream of the at least one guide RNA coding sequence.
Embodiment 13. The vector of Embodiment 12, wherein the polIII promoter is selected from a U6 and H1 promoter.
Embodiment 14. The vector of Embodiment 9, further comprising a regulatory control sequence
Embodiment 15. The vector of Embodiment 14, wherein the regulatory control sequence is a woodchuck hepatitis virus posttranscriptional regulatory element (WPRE).
Embodiment 16. The vector of Embodiment 9, wherein the vector is a recombinant adeno-associated virus vector (rAAV vector).
Embodiment 17. The vector of Embodiment 16, wherein the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
Embodiment 18. The vector of Embodiment 16 or 17, wherein the polynucleotide includes one or more inverted repeats (ITRs).
Embodiment 19. The vector of Embodiment 16, wherein the polynucleotide includes a poly A sequence.
Embodiment 20. The vector of Embodiment 9, wherein the polynucleotide is engineered to be expressed in a cell.
Embodiment 21. The vector of Embodiment 9, wherein the vector is a lentiviral vector, a gammaretroviral vector, or a herpes simplex viral vector.
Embodiment 22. The vector of Embodiment 9, wherein the vector comprises a split dCas9 vector system.
Embodiment 23. The vector of Embodiment 9, wherein the vector comprises a nucleic acid encoding a dCas9 having a sequence as set forth in SEQ ID NO:2.
Embodiment 24. The vector of Embodiment 9, wherein the vector comprises a nucleic acid encoding a KRAB sequence of SEQ ID NO:7.
Embodiment 25. The vector of Embodiment 22, wherein the split vector system comprises a vector sequence selected from SEQ ID NO: 3, 4 and 10.
Embodiment 26. A zinc-finger repressor construct comprising an engineered zinc finger DNA-binding domain coupled to a transcription repressor, wherein the zinc finger DNA-binding domain comprises one to six zinc-finger sequences and wherein the zinc finger sequences bind to a target nucleic acid sequence in a gene encoding a gene product selected from the group consisting of TRPV1/2/3/4, P2XR3, TRPM8, TRPA1, P23X2, P2RY, BDKRB1/2, Hlr3A, ACCNs, TRPV4, TRPC/P, ACCN1/2, SCN1/3/8A/9A, SCN10A, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited.
Embodiment 27. The zinc-finger repressor construct of Embodiment 26, wherein the target nucleic acid sequence is a sequence set forth in Table 2.
Embodiment 28. The zinc-finger repressor construct of Embodiment 26, wherein the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Embodiment 29. A polynucleotide encoding the zinc-finger repressor construct of Embodiment 26, 27 or 28.
Embodiment 30. The polynucleotide of Embodiment 29, wherein the polynucleotide is codon optimized for expression in a human cell.
Embodiment 31. A vector containing the polynucleotide of Embodiment 29.
Embodiment 32. The vector of Embodiment 31, wherein the polynucleotide is operably linked to a promoter.
Embodiment 33. The vector of Embodiment 32, wherein the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/camlodulin-dependent protein kinase II promoter, a tubulin alpha I promoter, a neuron-specific enolase promoter and a glial fibrillary acidic protein (GFAP) promoter.
Embodiment 34. The vector of Embodiment 31, further comprising a regulatory control sequence.
Embodiment 35. The vector of Embodiment 34, wherein the regulatory control sequence is a woodchuck hepatitis virus posttranscriptional regulatory element (WPRE).
Embodiment 36. The vector of Embodiment 31, wherein the vector is a recombinant adeno-associated virus vector (rAAV vector).
Embodiment 37. The vector of Embodiment 36, wherein the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
Embodiment 38. The vector of Embodiment 31, wherein the polynucleotide includes one or more inverted repeats (ITRs).
Embodiment 39. The vector of Embodiment 31, wherein the polynucleotide includes a poly A sequence.
Embodiment 40. The vector of Embodiment 31, wherein the nucleic acid is engineered to express the one or more components in a cell.
Embodiment 41. The vector of Embodiment 31, wherein the vector is a lentiviral vector, a gammaretroviral vector, or a herpes simplex viral vector.
Embodiment 42. The vector of Embodiment 31, wherein the vector comprises a nucleic acid encoding a KRAB sequence of SEQ ID NO:7.
Embodiment 43. An epigenetic-based method to treat or manage chronic pain in a subject comprising administering an effective amount of a complex of Embodiment 1 or a construct of Embodiment 26.
Embodiment 44. An epigenetic-based method to treat or manage pain in a subject in need thereof, comprising administering an effective amount of a zinc finger-repressor construct and/or a dCas9-repressor domain complex to the subject, wherein dCas9 is catalytically inactivated Cas9 that does not cleave DNA but maintains its ability to bind to the genome via a guide-RNA (gRNA).
Embodiment 45. The method of Embodiment 44, wherein the pain is selected from neuropathic pain, nociceptive pain, allodynia, inflammatory pain, inflammatory hyperalgesia, neuropathies, neuralgia, diabetic neuropathy, human immunodeficiency virus-related neuropathy, nerve injury, rheumatoid arthritic pain, osteoarthritic pain, burns, back pain, eye pain, visceral pain, cancer pain, bone cancer pain, migraine pain, pain from carpal tunnel syndrome, fibromyalgia pain, neuritis pain, sciatica pain, pelvic hypersensitivity pain, pelvic pain, post herpetic neuralgia pain, post-operative pain, post-stroke pain, and menstrual pain.
Embodiment 46. The method of Embodiment 44, wherein in the pain is associated with a disease or disorder selected from the group consisting of neuropathic peripheral neuropathy, diabetic neuropathy, post herpetic neuralgia, trigeminal neuralgia, back injury, cancer neuropathy, HIV neuropathy, limb loss, carpal tunnel syndrome, stroke, alcoholism, hypothyroidism, uremia, multiple sclerosis, spinal cord injury, Parkinson's disease, and epilepsy.
Embodiment 47. The epigenetic method of Embodiment 44, wherein the method is used to treat a subject with chronic pain.
Embodiment 48. The epigenetic method of Embodiment 44, wherein the zinc finger-repressor construct comprises a repressor domain selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Embodiment 49. The epigenetic method of Embodiment 48, wherein the repressor domain comprises KRAB.
Embodiment 50. The epigenetic method of Embodiment 44, wherein the zing finger-repressor construct binds to a target of Table 2.
Embodiment 51. The epigenetic method of Embodiment 44, wherein the dCas9-repressor domain complex comprises a repressor domain selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).
Embodiment 52. The epigenetic method of Embodiment 51, wherein the repressor domain comprises KRAB.
Embodiment 53. The epigenetic method of Embodiment 44, wherein the dCas9-repressor domain construct comprises a guide RNA spacer sequence having a sequence selected from SEQ ID NOs:11-106 and 107.
Embodiment 54. The epigenetic method of Embodiment 44, wherein the zinc finger-repressor construct and/or the dCas9-repressor domain construct provides for non-permanent gene repression of a voltage gated sodium channel.
Embodiment 55. The epigenetic method of Embodiment 54, wherein the voltage gated sodium channel is selected from NaV1.7, NaV1.8, and NaV1.9.
Embodiment 56. The epigenetic method of Embodiment 55, wherein the voltage gated sodium channel is NaV1.7.
Embodiment 57. The epigenetic method of Embodiment 44, wherein the zinc finger-repressor construct and/or the dCas9-repressor domain construct is packaged and delivered by a recombinant virus.
Embodiment 58. The epigenetic method of Embodiment 57, wherein the recombinant virus is an adenovirus, gammaretrovirus, adeno-associated virus (AAV), herpes simplex virus (HSV) or lentivirus.
Embodiment 59. The epigenetic method of Embodiment 57, wherein the recombinant virus is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
Embodiment 60. The epigenetic method of Embodiment 44, wherein the zinc finger-repressor construct and/or the dCas9-repressor domain construct is administered intravenous, intraperitoneal, intrathecal, intraganglionic, intraneural, intracranial or intramuscular.

## Claims

1. A zinc-finger repressor construct comprising an engineered zinc finger DNA-binding domain coupled to a transcription repressor, wherein the zinc finger DNA-binding domain comprises one to six zinc-finger sequences and wherein the zinc finger sequences bind to a target nucleic acid sequence in a gene encoding a gene product wherein the gene product is:
SCN9A; or
SCN10A; or
selected from the group consisting SCN1/3/8A, TRPV1/2/3/4, P2XR3, TRPM8, TRPA1, P23X2, P2RY, BDKRB1/2, Hlr3A, ACCNs, TRPV4, TRPC/P, ACCN1/2, SCN11A, KCNQ, BDNF, OPRD1/K1/M1, CNR1, GABRs, TNF, PLA2, IL1/6/12/18, COX-2, NTRK1, NGF, GDNF, TNF, LIF, CCL1, CNR2, TLR2/4, P2RX47, CCL2, CX3CR1, BDNF, NR1/2, GR1A1-4, GRC1-5, NK1R, CACNA1A-S, and CACNA2D1, wherein expression of the gene product is inhibited.

2. The zinc-finger repressor construct of claim 1, wherein the zinc-finger repressor comprises a nuclear localization signal (NLS), a linker, or both.

3. The zinc-finger repressor construct of claim 1, wherein the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B, DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).

4. The zinc-finger repressor construct of claim 1, wherein the transcriptional repressor domain is a KRAB domain.

5. A polynucleotide encoding one or more components of the zinc-finger repressor construct of claim 1.

6. A vector comprising a polynucleotide of claim 5, preferably wherein the polynucleotide is operably linked to a promoter.

7. The vector of claim 6, wherein the promoter is selected from the group consisting of a human cytomegalovirus (CMV) promoter, a CAG promoter, a Rous sarcoma virus (RSV) LTR promoter/enhancer, an SV40 promoter, a EF1-alpha promoter, a CMV immediate/early gene enhancer/CBA promoter, a Nav1.7 promoter, a Nav1.8 promoter, a Nav1.9 promoter, a TRPV1 promoter, a synapsin promoter, a calcium/calmodulin-dependent protein kinase II promoter, a tubulin alpha I promoter, a neuron-specific enolase promoter and a glial fibrillary acidic protein (GFAP) promoter.

8. The vector of claim 6, further comprising a regulatory control sequence, preferably wherein the regulatory control sequence is a woodchuck hepatitis virus posttranscriptional regulatory element (WPRE).

9. The vector of claim 8, wherein the vector is a recombinant adeno-associated virus vector (rAAV vector), preferably wherein the rAAV is selected from the group consisting of AAV1, AAV1(Y705+731F+T492V), AAV2, AAV2(Y444+500+730F+T491V), AAV3, AAV3(Y705+731F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV), optionally wherein the polynucleotide includes one or more inverted repeats (ITRs), or wherein the polynucleotide includes a poly A sequence.

10. The vector of claim 6, wherein the polynucleotide is engineered to be expressed in a cell, or wherein the vector is a lentiviral vector, a gammaretroviral vector, or a herpes simplex viral vector, or wherein the vector comprises a nucleic acid encoding a KRAB sequence of SEQ ID NO:7.

11. A zinc-finger repressor construct of claim 1, for use in treating or managing a chronic pain in a subject.

12. The zinc finger-repressor construct for use according to claim 11, wherein the pain is selected from neuropathic pain, nociceptive pain, allodynia, inflammatory pain, inflammatory hyperalgesia, neuropathies, neuralgia, diabetic neuropathy, human immunodeficiency virus-related neuropathy, nerve injury, rheumatoid arthritic pain, osteoarthritic pain, burns, back pain, eye pain, visceral pain, cancer pain, bone cancer pain, migraine pain, pain from carpal tunnel syndrome, fibromyalgia pain, neuritis pain, sciatica pain, pelvic hypersensitivity pain, pelvic pain, post herpetic neuralgia pain, post-operative pain, post-stroke pain, and menstrual pain, or wherein in the pain is associated with a disease or disorder selected from the group consisting of neuropathic peripheral neuropathy, diabetic neuropathy, post herpetic neuralgia, trigeminal neuralgia, back injury, cancer neuropathy, HIV neuropathy, limb loss, carpal tunnel syndrome, stroke, alcoholism, hypothyroidism, uremia, multiple sclerosis, spinal cord injury, Parkinson's disease, and epilepsy, or wherein the treatment is of chronic pain.

13. The zinc finger-repressor construct for use according to claim 11, wherein the transcription repressor is selected from the group consisting of mSin3 interaction domain (SID) protein, methyl-CpG-binding domain 2 (MBD2), MBD3, DNA methyltransferase (DNMT) 1 (DNMT1), DNMT2A, DNMT3A, DNMT3B, DNMT3L, retinoblastoma protein (Rb), methyl CpG binding protein 2 (Mecp2), Friend of GATA 1 (Fog1), regulator of MAT2 (ROM2), Arabidopsis thaliana HD2A protein (AtHD2A), lysine-specific demethylase 1(LSD1) and Krüppel-associated box (KRAB).

14. The zinc finger-repressor construct for use according to claim 11, wherein the transcriptional repressor domain is a KRAB domain.

15. The zinc finger-repressor construct for use according to claim 11, wherein said construct is packaged and delivered by a recombinant virus, preferably wherein the recombinant virus is an adenovirus, gammaretrovirus, adeno-associated virus (AAV), herpes simplex virus (HSV) or lentivirus, preferably wherein the recombinant virus is selected from the group consisting of AAV1, AAV1(Y705+731F+T492V), AAV2, AAV2(Y444+500+730F+T491V), AAV3, AAV3(Y705+731 F), AAV4, AAV5, AAV5(Y436+693+719F), AAV6, AAV6 (VP3 variant Y705F/Y731F/T492V), AAV7, AAV-7m8, AAV8, AAV8(Y733F), AAV9, AAV9 (VP3 variant Y731F), AAV10, AAV10(Y733F), AAV-ShH10, AAV11, AAV12 and a self-complementary vector (scAAV).
